# EUROPEAN PATENT APPLICATION

(11) **EP 2 033 656 A1**
(43) Date of publication of application: **11.03.2009**
(21) Application number: 07115641.8
(22) Date of filing: 04.09.2007
(51) Int. Cl.: A61K 38/28

(54) **Satiety-modulating compositions for oral application**

(71) Applicant: Stichting Top Institute Food and Nutrition, 6709 PA Wageningen (NL)
(72) Inventor: Harthoorn, Leunis, Forrinus, 3446 AG Woerden (NL); Schipper, Raymond, Godfried, 6521 KB Nijmegen (NL); Vingerhoeds, Monique, Henriëtte, 3706 TG Zeist (NL)
(74) Representative: Swinkels, Bart Willem

(57) **Abstract**

The invention pertains to the use of one or more peptides for the manufacture of a preparation for locally modulating satiety of a mammalian subject within the oral cavity of a mammalian subject, wherein the one or more peptides are selected from the group consisting of gastrointestinal and pancreatic peptide hormones, and salivary peptides. The invention also pertains to compositions for such use. The one or more peptides preferably exhibit a satiety correlation coefficient |r| in the range of 0.70 - 0.95 from SELDI-TOF-MS analysis on saliva.

## Description

### Field of the invention

The current invention relates to methods and means for screening peptides responsible for inducing satiety from saliva, to compositions comprising or consisting of one or more of such peptides, and the use of these compositions for manipulating a state of hunger or appetite, or satiety (and/or satiation) in a mammal. Such compositions can, therefore, be used to treat overweight or overweight related diseases, prevent weight gain, induce weight loss, for slimming etc.

### Background of the invention

Food consumption in mammals is under control of a complex interplay between mechanisms of the central nervous system and the peripheral body. In general the brain senses peripheral signals from the gastrointestinal tract, pancreas, and adipose tissue that reflect the body's nutritional state, and consequently employs several pathways to anticipate and adjust food intake and energy expenditure. Hence mammalian eating behavior is a complex interplay between hunger or appetite and satiety. The opposite of hunger and appetite is satiety, which is the physiological and psychological experience of "fullness" that comes after eating and/or drinking.

Health concerns nowadays focus on methods to prevent overweight and obesity. Presently, weight control is attempted through diets and prescription medication. However, diets generally do not provide a healthy nutritional balance and are difficult to accommodate in modem lifestyles. Drugs often have considerable risks associated with their use, and their administration is not preferred over longer time periods.

Satiety agents may play a role in a search for a safe yet effective method to control weight. A suitable class is formed from bio-markers, which are substances, and derivatives from central and peripheral processes, which relate systematically to food consumption, individual energy balance, and subjectively scored ratings for appetite. These bio-markers provide information about the metabolic physiology and nutritional state of the individual, and may be used as profiling tool for understanding the regulation of food intake and energy balance in relation to overweight and obesity (see e.g. De Graaf, C. et al. "Bio-markers of satiation and satiety"J. Am. J. Clin. Nutr. 2004, 79, 946-961).

One of the most accessible and valuable fluids for profiling bio-markers in particular is mammalian saliva. Saliva is secreted from exocrine glands and is suffused with lots of peptides and proteins. On the one hand, human saliva has multiple essential functions in relation to oral and general health, as well as to food processing and digestion that takes place in the upper parts of the gastrointestinal tract. Local synthesis and secretion of substances by the salivary glands is, thus, essential in maintaining a healthy oral environment and breakdown of for instance food starches. On the other hand, since saliva and in particular parotid saliva and also gingival crevicular fluids of the perodontium, represents an ultrafiltrate of the blood and contains many blood-born factors, it is a potential source of novel diagnostic markers and targets.

However, up to present no bio-markers have been identified to directly monitor perceived satiety in mammalian saliva. Aydin, S. et al. "A comparison of leptin and ghrelin levels in plasma and saliva of young healthy subjects" Peptides 2005, 26, 647-652 investigate the correlation between overnight-fasting levels of leptin and ghrelin in saliva and plasma, using ELISA or RIA assays. It suggests that measurement of ghrelin and leptin in saliva may be an acceptable alternative to plasma sampling for diagnostic fluid and predicts disease progression. However, Aydin et al. is silent on a direct correlation between the level of specific bio-markers in saliva, in particular stimulated saliva or parotid saliva, and the perceived satiety status in a subject, nor does it mention to apply these bio-markers in weight control.

Recently, Weinberger, S. R. et al. "Surface-enhanced laser desorption-ionization retentate chromatography mass spectrometry (SELDI-RC-MS): a new method for rapid development of process chromatography conditions." J. Chromatogr. B Analyt. Technol. Biomed. Life Sci. 2002, 782, 307-316 introduced surface-enhanced laser desorption/ionization time-of-flight (SELDI-TOF) mass spectrometry using ProteinChips). US-A-2004/0033613 successfully applies SELDI-TOF-MS in the field of diagnostic proteomics to detect several disease-associated proteins and protein expression patterns in a variety of biological tissues and body fluids, including saliva. Similarly, Schipper, R. G. et al. "SELDI-TOF-MS of saliva: methodology and pretreatment effects." J. Chromatogr. B Analyt. Technol. Biomed. Life Sci. 2007, 15, 847, 45-53, and Schipper, R. G. et al. "Salivary protein/peptide profiling with SELDI-TOF-MS"Ann NY Acad Sci 1098 (2007) 498-503, found that SELDI-TOF-MS allows for detection of specific bio-markers in human saliva. However, both do not discuss possibilities on modulating satiety.

Hence, there is a need to develop and provide pharmaceutical or nutritional or neutraceutical compositions, such as food or feed products, food supplements, meal replacement products and other products to be used in weight control plan, that have an improved and immediate satiety and/or satiation effect.

### Summary of the invention

It is an objective of the invention to provide pharmaceutical and nutritional compositions which manipulate mammalian (human or animal) eating behavior, either by inducing a feeling of hunger or appetite, or a feeling of satiety and/or satiation.

Thereto, as a first step, the inventors have found methods and means allowing for the detection of peptides in saliva that have a direct correlation with perceived satiety in a mammalian subject. These allow for a rapid and reliable screening of the level of satiety in subject, or changes in the level of satiety, as well as for the satiety-inducing properties of compounds, compositions, food supplements, food products and nutraceuticals.

These screening methods also allow for an easy, cost-effective and rapid screening of satiety levels or change in satiety levels in a subject without having to collect blood samples at multiple times, which is unpleasant for the subject under study, more laborious and expensive. In addition blood sampling is known to cause a mild stress response in certain individuals. A stress response may influence feelings of hunger and satiety as well as the bio-marker levels under study, which is highly disadvantageous and may be circumvented by the method and means provided by the current invention.

The screening method provided for makes it possible to select peptides that modulate satiety locally, i.e. within the oral cavity of the mammalian subject. Advantageously, these peptides show immediate action in the oral cavity, without requiring the peptides to enter the blood stream, the stomach and the intestine of the mammalian subject. From this screening method it was derived that thirty masses demonstrated a strong correlation with all subjectively scored satiety ratings, while seven peptides were significantly correlated to body mass index (BMI).

The inventors have now found that the screening method allows for a selection of peptides which can advantageously be included in pharmaceutical and/or food compositions and supplements, to provide the consumer with a certain level of satiety, depending on the satiety-inducing properties of the peptide(s). These compositions and supplements may be used in weight control, in particular in cases of overweight and obesity. Since the screening method focuses on the availability and effect of such peptide bio-markers in the oral cavity, it is possible to select peptides which have satiety-modulating effects and/or a satiation inducing effect upon release in the oral cavity. Hence, the peptide comprising food supplements particularly strived for need not be swallowed.

### Detailed description of the invention

### Screening method

In a first aspect the present invention thus pertains to a method for determining the satiety status of a mammalian subject comprising (a) determining in a sample of saliva from the subject the levels of one or more peptides; and (b) correlating said levels with the satiety status, i.e. determining the satiety status of the mammalian subject there from.

The satiety status of a human subject may be defined as set out in the examples section by answering a questionnaire on satiety, desire to eat, appetite, fullness, hunger, thirst, general well being, eating behavior, food preference or selection etc. When the method of the invention is applied to non-human mammals, satiety may be measured by monitoring ethology: the eating behavior or pattern, food searching behavior, food intake, food preferences etc.

The screening method may preferably be carried out on saliva obtained from mammalian subjects, most preferably on human subjects although the method may also be adapted for use in various animals, bovine, canines, cats, horses, sheep, goats, pigs and rodents. It may be readily carried out on whole saliva which can be easily sampled, but is more preferably carried out on parotid saliva or stimulated saliva that is rich in parotid saliva, in order to obtain optimal results for determining satiety.

The screening method comprises the detection of at least one peptide(s) having oral activity, and that may increase or decrease upon food intake of the mammal after a period of fasting / food deprivation or after a change in diet / food supply. Preferably the screening method comprises the sequential or simultaneous detection of 2, 3, 4, 5, 6, 7, 8, 9, 10 or more peptides that increase or decrease with changes in the level of satiety in the mammal, in order to increase the sensitivity and consistency/reproducibility of the method. Most preferably, the screening method comprises the detection of 2 to 6 satiety peptides, with at least 1 up-regulated peptide and 1 down-regulated peptide in saliva samples, as an optimal number yielding sufficient sensitivity and practicality. The peptides according to the invention that correlate with satiety may correlate positively or negatively, i.e. may increase or decrease in saliva with induction of experiencing satiety in the subject.

The above method may comprise multiple sampling and measuring, e.g. before/after food intake, to track any changes in the levels of satiety peptides. Alternatively, the method involves a single measurement, comparing the figures thus obtained with a database of peptides. Preferably the method comprises the sequential detection of peptide levels in saliva obtained from the subject under two different conditions. The two different conditions and comparison of the two obtained mass spectra from saliva samples may for instance reflect the peptide spectrum before and after uptake of a substance, a composition, a food product, a medicament or before and after the subject has undergone a certain treatment, all of which may alter the perceived level of satiety of the subject.

Changes in the level of satiety peptides in saliva may be determined as the fold increase/decrease after food intake and induction of satiety, or alternatively as the relative increase or decrease as compared to peptides in the saliva sample that remain relatively constant and/or are unaffected by satiety or food intake. Satiety peptides may also be measured as increases/decreases in absolute level (i.e. concentration) in saliva. In addition or as an alternative, the satiety peptides of the invention may also be correlated to one another; i.e. the increase of one or more satiety peptides in saliva may be determined by a correlation to a relative decrease in one or more (other) satiety peptides, or as the ratio of two or more peptides.
The step of determining the peptide peptides in a sample of saliva may be carried out using Surface-Enhanced Laser Desorption/Ionization Time-Of-Flight Mass Spectrometry (SELDI-TOF MS).

SELDI-TOF-MS combines matrix-assisted laser-desorption/ionization time-of-flight mass spectrometry (MALDI-TOF-MS) to surface chromatography. It utilizes sample chips which display various kinds of chemically enriched and active surfaces that bind protein and peptide molecules based on established principles such as ion exchange chromatography, metal ion affinity, or hydrophobic affinity. The subset of proteins and peptides retained to a certain surface can be varied by changing the properties (e.g. pH, salt concentration) of the binding buffer and the stringency of the washing steps for removal of unbound or weakly bound molecules and salts, which can interfere with the analysis. Protein detection is then accomplished by TOF-MS. This technology advantageously enables rapid and high-throughput detection of critical proteins and peptides directly from complex biological fluids without labor- intensive pre-processing and requiring only minute amounts of sample.

The inventors studied the usefulness of the technique by investigating the characteristics of the human saliva proteome in a food intake-related context. In a longitudinal setting, subjects were exposed to a lunch-induced hunger to satiety shift and saliva samples were collected before and after consumption. Saliva samples were then analyzed by SELDI-TOF-MS, which allowed tracking changes of its composition based on about 190 of the most abundant masses. Thirty masses demonstrated a strong correlation with all subjectively scored satiety ratings, while 7 peaks were significantly correlated to body mass index (BMI). More details are given in the accompanying examples.

In another embodiment, the step of determining the level of satiety peptides in saliva may be carried out by applying an antibody-based assay, i.e. an immuno-assay. For the peptides at dispute, in particular the preferred gastrointestinal and pancreatic peptide hormones, monoclonal antibodies are commercially available from various suppliers all of which may be successfully applied, taking into account the manufacturers recommendations for use. Suitable immuno-assays for determining the level of a satiety peptide in saliva comprise various antibody-based assays known in the art and for instance described in Current Protocols in Immunology, J.E. Coligan et al, Wiley Interscience 2006. Suitable assays comprise ELISA, RIA, protein arrays, gel-shift and immunoprecipitation assays. Most preferably the method of the invention is carried out using a solid carrier comprising one antibody specific for one or more saliva satiety peptide(s) of the invention. A solid carrier for carrying out the method of the invention comprises one or more antibodies or functional fragments thereof specific for gastrointestinal and/or pancreatic peptide hormones.
In another aspect the current invention provides a solid carrier, preferably in the form of a (micro)-array, protein chip, test-dipstick or test-tube, suitable for carrying out the method of the invention, the carrier comprising one or more monoclonal or polyclonal antibodies or functional fragments thereof such as Fab, Fab2, scFv and VHH, that are specific for a satiety peptide present in saliva. Preferably the solid carrier of the invention comprises antibodies with specificity for several target peptides in distinct areas on the carrier in order to facilitate the sequential or simultaneous detection of at least 2, 3, 4, 5, 6, 7, 8, 9, 10 or more peptides that increase or decrease with changes in the level of satiety in the mammal. The solid carrier of the invention preferably comprises antibodies or functional antibody fragments which are specific for a gastrointestinal and/or pancreatic peptide hormone.

The present invention also refers to a method for identifying a peptide correlating with a level of satiety in a mammal using SELDI-TOF MS, wherein the method comprises: i) subjecting a sample of saliva from the mammal to SELDI-TOF mass spectrometry, ii) correlating the peaks / molecular masses in the obtained spectrum with a state of satiety in the mammal and iii) determining the peptide identity of the satiety correlated peaks in the SELDI-TOF mass spectrum.
This method may form an integral part of the above determination of the satiety status of a mammalian subject, or it may be part of the formation of a data base of satiety peptides, which in turn may be applied for the determination of the satiety status of a mammalian subject.

The method for determining the satiety status of a mammalian subject is also applicable to the screening and the development of novel food products and diets that induce desired levels of satiety. The method may also be applied for screening compounds, compositions, food ingredients / nutrients, food products and nutraceuticals for their capability of inducing or reducing satiety in a mammal. The invention may particularly be used to study satiety inducing capabilities of various combinations of substances and food products. In addition, the identified satiety peptides may be applied to design a solid carrier comprising antibodies or functional fragments thereof that are specific for the satiety peptide identified, and which may be applied in carrying out the method of the invention.

### Satiety-modulating compositions and/or satiation-modulating compositions

In another aspect the present invention pertains to the use of one or more peptides for the manufacture of a preparation for locally modulating satiety and/or satiation of a mammalian subject within the oral cavity of a mammalian subject. The invention is particularly directed to the use of these peptides in food applications.

As in foregoing, "mammalian subject" favorably includes human beings, but also comprises various animals, such as bovine, canines, cats, horses, sheep, goats, pigs and rodents.

With "locally" it is understood that the peptide(s) present in the composition modulate(s) satiety locally, within the oral cavity, without the need for these peptides to pass on and enter the blood stream, the stomach and/or intestine. In view thereof, the preparation is meant to be administered orally; intravenous administration does not form part of the invention. It is stressed that the invention does not exclude the occurrence of the bio-marker peptides contained in the composition in other parts of the gastrointestinal tract, provided that these peptides have their action in the oral cavity. The above screening method is a good indicator to establish this. For this purpose, it is especially desired that the peptides and peptide hormones have an oral receptor.

The degree of satiety may vary between hunger and appetite on the one side, and satiety, i.e. the physiological and psychological experience of "fullness" that comes after eating and/or drinking, on the opposite side. Hence, hunger and satiety form the respective low and high ends of the "satiety scale". Thus, "modulating satiety" may involve either inducing or reducing satiety, the latter corresponding to a feeling of hunger or appetite. "Satiation" or "enhanced satiation" refers to earlier meal termination due to an earlier feeling of fullness. In the context of the invention, "satiety" and "satiation" are used interchangeably.
The peptides are preferably those screened peptides found in the SELDI-TOF MS screening method.

Throughout the description, the terms "peptides" and "peptide bio-markers" are used interchangeably, and are meant to include peptide modifications, such as glycosylations, phosphorylations, O-octanoylations and/or O-decanoylations. Here below, the term "peptide" includes proteins and peptide hormone. As in the art, the peptides referred to are differentially present in samples from multiple subjects or from a test subject at different conditions. Therein, "differentially present" refers to differences in the quantity, frequency or modification of a peptide.

The peptides are preferably selected from the group consisting of gastrointestinal and pancreatic peptide hormone(s) and salivary peptides. With "salivary peptides" it is understood peptides which are naturally present in saliva. It preferably concerns salivary proteins and peptides ranging from 2 to 100 kDa, more preferably low-mass saliva proteins/peptides (< 10 kDa).

The peptides are more selected from the group of peptides found in the aforementioned SELDI-TOF-MS-based satiety screening method, and presented in table 1, in which 30 peptides show to have a high level of correlation with satiety. The satiety-modulating effect of these peptides is reflected in the SELDI-TOF-MS satiety correlation coefficient |r_{satiety}|. It is found that is especially preferred to select a salivary peptide which scores a SELDI-TOF-MS satiety correlation coefficient |r_{satiety}| in the range of 0.70 - 0.95, preferably at least 0.75, especially at least 0.80. The numbers in table 1 are derived from SELDI-TOF-MS analysis on human saliva.

In addition, the composition may further comprises PYY3-36, since it appeared to be found in the saliva satiety screening method, despite its relatively low satiety correlation coefficient.

The use of the peptides either as a satiety-reducing or satiety-inducing agent can be directly derived from table 1: The sign of the correlation coefficient r_{satiety} corresponds to its satiety- or hunger-inducing effect: a negative value corresponds to a satiety-reducing effect, a positive value is associated with a satiety-inducing effect.

**Table 1 - Potential satiety-affecting peptides according to SELDI-TOF-MS**

| **peptide** | **peak** | ***m*/*z*** | ***r* satiety** | ***r* desire** | ***r*fullness** | ***r* hunger** |
|---|---|---|---|---|---|---|
| neuropeptide SF | A2 | 1361 | -0.73^{c} | 0.73^{c} | -0.71^{c} | 0.67^{b} |
| kisspeptin-14 | B3 | 1737 | -0.76^{c} | 0.75^{c} | -0.76^{c} | 0.69^{c} |
| - (unknown) | B6 | 1951 | -0.82^{c} | 0.76^{c} | -0.84^{c} | 0.78^{c} |
| insulin-like 3A chain | D6 | 2780 | -0.83^{c} | 0.84^{c} | -0.83^{c} | 0.85^{c} |
| early placenta insulin-like peptide A chain | | | | | | |
| P-beta | D8 | 2874 | -0.81^{c} | 0.78^{c} | -0.79^{c} | 0.70^{c} |
| ghrelin-28 (O-octanoylated) | E8 | 3370 | -0.72^{c} | 0.64^{b} | -0.73^{c} | 0.64^{b} |
| neutrophil defensin-2 | | | | | | |
| hypothetical protein C21 orf89 | | | | | | |
| insulin B chain | E9 | 3438 | -0.80^{c} | 0.75^{c} | -0.78^{c} | 0.74^{c} |
| histatin-2 | | | | | | |
| glucagon | F1 | 3480 | -0.70^{c} | 0.64^{b} | -0.72^{c} | 0.63^{b} |
| C-flanking peptide of NPY | | | | | | |
| Peptide YY (3-36) | F8 | 4051 | 0.46^{a} | -0.39 | 0.50^{a} | -0.36 |
| BAX protein, cytoplasmic isoform γ | H1 | 4688 | 0.82^{c} | -0.74^{c} | 0.85^{c} | -0.70^{c} |
| urocortin | | | | | | |
| Cyt c oxidase PP VIII-liver/heart, mit. precursor | H7 | 4899 | 0.72^{c} | -0.64^{b} | 0.74^{c} | -0.63^{b} |
| thymosin beta-10 | | | | | | |
| thymosin beta-4 | H8 | 4919 | 0.72^{c} | -0.63^{b} | 0.74^{c} | -0.59^{b} |
| - (unknown) | H9 | 4943 | 0.78^{c} | -0.70^{c} | 0.80^{c} | -0.70^{c} |
| beta-defensin 126 | I1 | 4964 | 0.72^{c} | -0.64^{b} | 0.75^{c} | -0.67^{b} |
| somatomedin B | I2 | 5005 | 0.70^{c} | -0.59^{b} | 0.71^{c} | -0.57^{b} |
| - (unknown) | I4 | 5121 | 0.74^{c} | -0.65^{b} | 0.77^{c} | -0.64^{b} |
| granulin-5 | J6 | 5681 | -0.71^{c} | 0.70^{c} | -0.69^{c} | 0.72^{c} |
| small inducible cytokine A22 | M5 | 8098 | 0.73^{c} | 0.66^{b} | -0.75^{c} | 0.68^{c} |
| glicentin | | | | | | |
| IL-8(9-77) | | | | | | |
| neutrophil-activating peptide 2 (74) | | | | | | |
| - (unknown) | M6 | 8122 | -0.80^{c} | 0.74^{c} | -0.83^{c} | 0.77^{c} |
| - (unknown) | M9 | 8334 | -0.75^{c} | 0.69^{c} | -0.80^{c} | 0.73^{c} |
| basic nuclear protein HPS2 | N3 | 8725 | -0.78^{c} | 0.73^{c} | -0.83^{c} | 0.74^{c} |
| CSL-type zinc finger-containing protein 1 | | | | | | |
| apolipoprotein C-II | N4 | 8922 | -0.70^{c} | 0.62^{b} | -0.72^{c} | 0.68^{c} |
| small inducible cytokine A8 | | | | | | |
| interleukin-8 | | | | | | |
| activation peptide | | | | | | |
| secreted Ly-6/uPAR-related protein 1 | | | | | | |
| ubiquitin-fold modifier 1 | | | | | | |
| keratin-associated protein 19-6 | N6 | 9123 | -0.92^{c} | 0.88^{c} | -0.91^{c} | 0.82^{c} |
| U6 snRNA-associated Sm-like protein LSm6 | | | | | | |
| Saposin D | | | | | | |
| Down syndrome critical regulatory protein 10 | N8 | 9281 | -0.76^{c} | 0.69^{c} | -0.77^{c} | 0.71^{c} |
| keratin-associated protein 7-1 | | | | | | |
| NADH dehydrogenase 1 α subcomplex subunit 3 | | | | | | |
| ATPase inhibitor | N9 | 9507 | -0.90^{c} | 0.87^{c} | -0.92^{c} | 0.86^{c} |
| hypothetical protein C9orf27 | | | | | | |
| keratin-associated protein 17-1 | | | | | | |
| Late cornified envelope protein 3E | | | | | | |
| stannin | | | | | | |
| retinal rod rhodopsin-sensitive cGMP PDE γ-subunit | O1 | 9645 | -0.71^{c} | 0.66^{b} | -0.70^{c} | 0.58^{b} |
| COMM domain-containing protein 6 | | | | | | |
| thioredoxin domain-containing protein C9orf121 | T5 | 17613 | 0.77^{c} | -0.70^{c} | 0.79^{c} | -0.69^{c} |
| HERV-K_3q21.2 provirus ancestral Gag polyprotein | | | | | | |
| 60S ribosomal protein L29 | | | | | | |
| protein SYS1homolog | | | | | | |
| zinc finger HIT domain-containing protein 3 | | | | | | |
| ADP-ribosyl cyclase 2 | U7 | 30081 | 0.71^{c} | -0.66^{b} | 0.71^{c} | -0.68^{c} |
| methyltransferase-like protein 6 | | | | | | |
| THAP domain-containing protein 8 | | | | | | |
| ubiquinone biosynthesis protein COQ9 | U8 | 30941 | 0.73^{c} | -0.65^{b} | 0.74^{c} | -0.63^{b} |
| homeobox protein MSX-1 | | | | | | |
| SPRY domain-containing SOCS box protein 1 | | | | | | |
| tissue-type plasmonigen activator chain A | | | | | | |
| testis expressed sequence 264 protein | | | | | | |
| transcription cofactor vestigial-like protein 4 | | | | | | |
| NG,NG-dimethylarginine methylaminohydrolase 1 | U9 | 30991 | 0.70^{c} | -0.64^{b} | 0.69^{c} | -0.60^{b} |
| elongation factor 1-δ | | | | | | |

| | | | | | | |
|---|---|---|---|---|---|---|
| a: * *p* < 0.05; b: * *p* < 0.01; c: * *p* < 0.001 | | | | | | |

Gastrointestinal and pancreatic peptide hormones that are particularly suitable to be applied in the context of the invention comprise the following: insulin, insulin α-chain, insulin β-chain, insulin-like peptides 1-3, somatostatin-14 and -28, gastrin, cholecystokinin (CCK), secretin, motilin, vasoactive intestinal peptide (VIP), gastric inhibitory polypeptide, enteroglucagon, peptide YY (P-YY), pancreatic hormone, pancreatic icosapeptide, ghrelin, glucagon, glucagon-like peptide 1 (GLP-1), glucagon-like peptide 2 (GLP-2), glicentin, glucose-dependent insulinotropic polypeptide (GIP) and amylin.

Salivary peptides preferably comprise defensins, somatomedins, histatins and leptins. These peptides are preferably applied in combination with one or more of the aforementioned gastrointestinal and/or pancreatic peptide hormone(s). The most preferred salivary peptides are somatomedin B, neutrophil defensin-2, histatin-2 and beta-defensin 126, the levels of which can be readily determined in saliva and that show a high correlation to perceived satiety. In another embodiment it is preferred to use leptin, a peptide hormone synthesized and secreted by fat tissue, preferably in combination with the above mentioned group of gastrointestinal and pancreatic peptide hormones.
Of the above gastrointestinal and pancreatic peptide hormones and salivary peptides the ones for which oral receptors are known to exist are the most preferred candidates, especially if time over which an effect is to be established plays an important role, as is often the case. These include ghrelin, cholecystokinin (CCK) and leptin (Bohlender et al., 2003; Gröschl et al., 2005; Herness et al., 2002).

The proteins or peptides according to the invention comprise or consist of those provided in Table 5, and in SEQ ID NO: 1-96. Encompassed herein are also variants and fragments of the proteins/peptides or of the variants. "Variants" include any protein or peptide which comprises at least 70, 80, 90, 95, 98, 99% or more amino acid sequence identity to one or more of the peptides of SEQ ID NO: 1-96. Sequence alignment can be carrier out using for example the programs " Needle" or "Water" of EmbossWin (e.g. version 2.10.0), using default parameters. "Needle" uses the Needleman and Wunsch global alignment algorithm to align two sequences over their entire length, maximizing the number of matches and minimizes the number of gaps. Needle is preferably used when aligning sequences of similar lengths. For comparing sequence identity between sequences of dissimilar lengths, it is preferred that local alignment algorithms are used, such as the Smith Waterman algorithm (Smith TF, Waterman MS (1981) J. Mol. Biol 147(1);195-7), used e.g. in the EmbossWIN program "water". Default parameters for "needle" and "water" are gap opening penalty 10.0 and gap extension penalty 0.5, using the Blosum62 matrix for proteins.
"Fragments" of proteins or peptides (or of variants) include parts thereof, such as at least 5, 10, 15, 20, 30, or more contiguous amino acids.
Optionally, in determining the degree of amino acid similarity, the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to ser; Arg to lys; Asn to gln or his; Asp to glu; Cys to ser or ala; Gln to asn; Glu to asp; Gly to pro; His to asn or gln; Ile to leu or val; Leu to ile or val; Lys to arg; gln or glu; Met to leu or ile; Phe to met, leu or tyr; Ser to thr; Thr to ser; Trp to tyr; Tyr to trp or phe; and, Val to ile or leu.
The particularly preferred peptides comprise insulin-like peptide A chain, insulin B chain, ghrelin and glucagons, or similar forms thereof, in accordance with the above-given homology test.
Obviously, the invention is not limited to saliva as the source of these peptides. Hence, the terms "salivary peptides", and "gastrointestinal- and/or pancreatic hormone peptides" are not regarded binding in terms of their actual source. The peptides which are subject of the invention may be derived from all kinds of other (natural) sources. The skilled person will be determine those with the information given above, and with the aid of the sequence listings attached.
The peptides of the invention are preferably applied in a dosage in the range of 0.01 nM and 1 mM, preferably less than 0.1 mM, the specific dosages depending on the actual peptides used. As an example, satiety-reducing insulin alpha chain, insulin-like peptides 1 - 3 and glicentin, and satiety-inducing beta-defensin 126 and somatomedin B (insulin-like growth factor) are preferably applied in concentrations in the range of 1-300 nM, while satiety-reducing ghrelin-28 (O-octanoylated), insulin-beta chain and glucacon, and satiety-reducing neutrophil defensin-2 and histatin are typically used in higher amounts, e.g. 10 - 2000 nM. However, synergistic mixtures of the peptides may reduce the required amounts.

In yet another aspect the invention pertains to a composition for administration to the oral cavity of a mammalian subject, wherein the composition comprises a satiety-manipulating amount of one or more of the aforementioned gastrointestinal- and/or pancreatic hormone peptides, and/or salivary peptides.
In one preferred embodiment, the composition contains one or more salivary peptides.
Alternatively, the composition comprises one or more gastrointestinal- and/or pancreatic hormone peptides selected from the group consisting of insulin α chain, insulin β chain, insulin-like peptides 1-3, somatostatin, gastrin, secretin, motilin, vasoactive intestinal peptide (VIP), gastric inhibitory polypeptide, enteroglucagon, pancreatic hormone, pancreatic icosapeptide, ghrelin, glucagon, glucagon-like peptide 2 (GLP-2), glicentin, glucose-dependent insulinotropic polypeptide (GIP). Thus, mixtures of two, three, four, five, or more peptides according to the invention are included herein.
The composition is intended for oral use, and directed to achieve a local effect in the oral cavity of the mammalian subject. The composition is not saliva, although it may comprise processed saliva components. As mentioned above, the peptides are preferably obtained from a more processable source. Hence, the composition may conveniently be described as being compounded. Its fabrication involves an industrial processing step other than storage.
In line with the above, the term "satiety-modulating" is understood to comprise either "satiety-inducing" and "satiety-reducing". The actual effect is dependent on the choice of peptides. The effect of a "test" composition comprising or consisting of one or more peptides of the invention on satiety can be determined by scoring for example the period of fullness until the next meal. A satiety inducing effect is seen if the period of the feeling of fullness is significantly longer than in the controls, such as for example at least 1%, 2%, 3%, 5%, 10%, 20% longer, or more. In addition or alternatively, the satiety inducing effect can be determined by measuring the amount of food consumed (until a pleasant feeling of fullness is reached) during a meal that is taken after a certain period (e.g 4 hours) following use of the test composition. The same test can be done for satiety reducing peptides and compositions, whereby the feeling of fullness is significantly shorter after use of the composition (e.g. chewing or contact with the oral cavity) compared to controls.

An effect on satiation can be determined by e.g. scoring the time point of meal termination. A satiation inducing effect is seen if the amount of food or consumed calories at meal termination is significantly less than in the controls, such as for example at least 1%, 2%, 3%, 4%, 5%, 10% 20%, or more. When using the composition over a longer time period (several weeks or more), one can also score the body weight reduction or the body weight change compared to a control diet. Body weight of a subject being administered regular amounts of the test compositions (e.g. once daily, twice daily, or more) is preferably significantly controlled (reduced or less increased) compared to the control subjects. The same test can be done for satiation reducing peptides and compositions, whereby the time-point of meal termination is significantly longer after use of the composition (e.g. chewing or contact with the oral cavity) compared to controls.
In human subjects subjective ratings before and at certain time intervals after use of the composition(s) can be assessed (e.g. subjective hunger, fullness, satiety, desire to eat, etc.) using, for example, the Visual Analogue Scale (VAS), as known in the art (see e.g. Luscombe-Marsh et al., Am. J. Clin. Nutr. (2005) 81:762-772 or Moran et al., J. Clin. Endocrinol. Metabol. (2005)90:5205-5211).

The peptides can be synthesized by chemical peptide synthesis using standard methods. Alternatively, they can be isolated from natural sources.

A satiety-inducing composition is preferably free from peptides having a satiety correlation coefficient r_{satiety} <-0.70, more preferably <-0.65, while a satiety-reducing composition does preferably not contain any peptides having a satiety correlation coefficient r_{satiety} > 0.70, more preferably > 0.65. In one embodiment, the composition contains only those peptides having the same sign for their satiety correlation coefficient r_{satiety}, as obtained from SELDI-TOF-MS_{.}
In principle, the composition may be a pharmaceutical preparation. If so, it is intended for oral administration. However, it is preferred that the composition is a nutritional food or feed product, or a food or feed supplement. It may be in liquid, semi-solid or solid form.
Because of their desired oral activity, the peptides are preferably not encapsulated (unprotected), i.e. freely dispersed in the composition. This guarantees immediate release of the peptides in the oral cavity upon consumption, especially desired in for those peptides for which oral receptors exist.

### Meal replacement

In one embodiment the food product is a meal replacement or weight control product containing one or more of the aforementioned satiety-modulating peptides. The term "meal replacer" refers to a product which is intended to replace one or more conventional meals a day as part of a body weight loss or body weight management plan; they are of controlled calorie content and are generally eaten as a single product. The term also includes products which are eaten as part of a meal replacement weight loss or weight control plan, for example snack products which are not intended to replace a whole meal by themselves but which may be used with other such products to replace a meal or which are otherwise intended to be used in those plans; these products typically have a calorie content in the range of 50 - 150 kcal per serving. Generalized, the meal replacement or weight control product includes all foodstuffs which are to be swallowed.
Examples of meal replacement products include liquid products such as milk or soya-based drinks, soluble powders used to prepare those drinks and drinks prepared there from, bars, soups, cereal or noodle or pasta-based products, desserts such as rice puddings, custards, and the like and porridge and the like.
It is especially preferred that the meal replacement is a ready-to-drink liquid, a liquid produced from a soluble powdered product, a soup, a dessert, a bar, a cereal based or pasta based or noodle based product, or, a soluble powdered product.
The calorie content of the meal replacement is preferably in the range of from 50 kilocalories (kcals) to 600 kcals, more preferably 100 kcals to 500 kcals, most preferably 150 kcals to 450 kcals per serving.
Other food products intended to be used as part of a body weight loss or body weight control plan typically have fewer calories per serving (or per 100 g of product) than their non-diet' equivalents. The calorie content of these foods is deliberately restricted accordingly. Examples include the so-called low-calorie options of every day foods such as icecream, cream, cakes, puddings, yoghurt etc. Meal replacement products do not generally fall in this category as there may be no full calorie equivalent product and also it is necessary to provide a reasonable number of calories per meal replaced.
The amounts of protein, fat, carbohydrate and other ingredients in the food product will vary according to the product format of the composition and also, where required, according to national or regional legislation.
The edible composition will typically comprise proteinaceous material other than the satiety-modulating peptides. It is preferred that the product comprises at least 1wt% protein based on the weight of the product. Preferably the product comprises protein in an amount of from 1.5 to 25 wt%, preferably 2 to 20 wt%. The protein may be chosen from any source, including milk proteins, egg proteins, animal proteins and plant proteins. It is further preferred that the protein provides up to 75 % of the total calories of the product, more preferably between 10 % and 40 %, most preferably between 15 and 35 %.
The food product may comprise edible fats, preferably in an amount of up to 60 or 70 % by weight based on the weight of the product, more preferably from 0.5 to 30 or 35 wt%, most preferably from 0.75 to 20 wt% fat. Any suitable fat may be used for example, animal fats, vegetable fats, plant oils, nut oils, seed oils, or mixtures thereof. Saturated or unsaturated (mono-unsaturated and poly-unsaturated) fats may be used.
The food products may also comprise one or more carbohydrates, preferably in an amount of from 1 to 95 % by weight based on the weight of the product, more preferably 2 to 60 wt%, most preferably 5 to 50 wt%, such as 10 to 40 wt%. Any suitable carbohydrate may be used, for example sucrose, lactose, glucose, fructose, corn syrup, sugar alcohols, maltodextrins, starch, modified starch or mixtures thereof.
The food product may also comprise soluble or insoluble dietary fibres, for example in an amount of from 0.1 to 50 % by weight based on the weight of the product, preferably 0.5 to 20 wt%.
If the satiety-manipulating compositions form part of a diet, it is beneficial to include health-promoting ingredients into the composition, to provide a healthy nutritional balance. Typical health-promoting ingredients are vitamins, minerals and trace elements, summarized as micronutrients. Often, these nutrients are not present in adequate amounts in a diet to meet the daily requirements. The composition preferably comprises added vitamins and/or minerals, preferably selected from at least one of vitamins A, B1, B2, B3, B5, B6, B11, B12, biotin, C, D, E, H, and K and minerals calcium, magnesium, potassium, zinc, iron, iodine, manganese, molybdenum, phosphorous, selenium and chromium. The vitamins and/or minerals may be added by the use of vitamin premixes, mineral premixes and mixtures thereof or alternatively they may be added individually.

The food application is preferably one with long oral cavity residence times, to facilitate a long-lasting effect or maintenance of satiety modulation.

### Other food applications

In a preferred embodiment, the satiety-modulating peptide bio-markers of the invention are incorporated into a composition intended for use in the oral cavity without the need for swallowing. Hence, such compositions typically do not contain any caloric components, although this is not excluded. Typical examples are sprays, aerosols, toothpaste and chewing gum. Consumption of these types of food applications typically involves long-lasting oral cavity residence times and excellent release properties.
It is particularly preferred that the satiety-modulating peptide bio-markers are incorporated in a chewing gum or a toothpaste, most preferably a chewing gum. Especially where intended for satiety-inducing application, the use of chewing gum is a surprising one, since chewing is normally known to activate the digestive system. Therefore, conventional diets often teach to avoid chewing gum.

### Figure legends

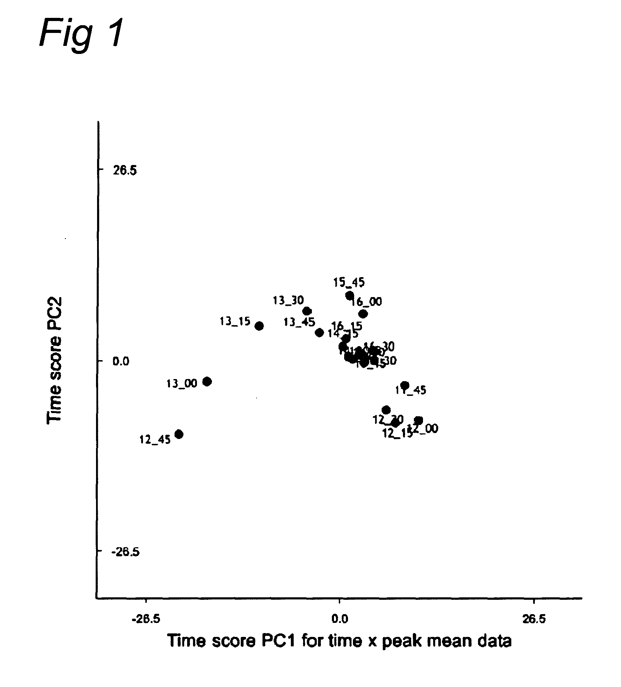

Figure 1: PC (principal component) scores for the time points for the 2 D model for PC1 and PC2. The scores of the time points appear to cluster in those before lunch, those immediately after lunch and a set consisting of the remaining time points.

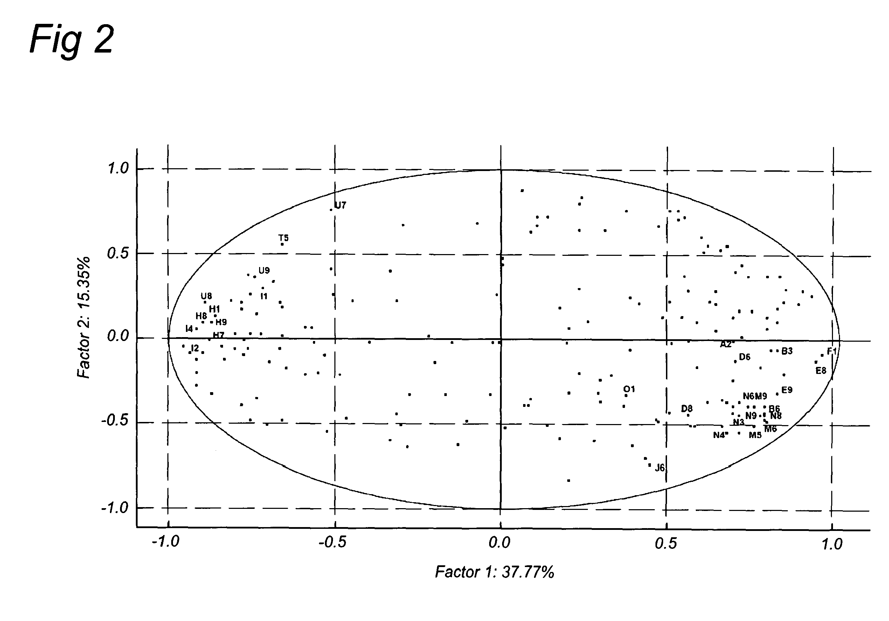

Figure 2: PC (principal component) scores of peptides and proteins (peak intensities). Twenty-nine coded dots are components significantly related to satiety. Their interrelationships show that 38% of the variation was accounted for in one dimension.

### Examples

### EXAMPLE 1 - Determining satiety status of human subjects with SELDI-TOF-MS

Healthy subjects, 9 males and 9 females, aged between 23 and 63 years were tested at the sensory research facilities of the Agrotechnology & Food Sciences Group, Wageningen, The Netherlands. The subjects were paid for their involvement, and gave written informed consent according to the guidelines of the ethical committee of Wageningen UR, Wageningen, The Netherlands. Anthropometrical measurements including weight and height were made according to the study protocol by trained personnel. BMI was calculated as weight divided by height squared. Each individual subject was tested alone in a quiet furnished room with appropriate ventilation and lighting. Testing took place over a 4¾-hour session, from 11:45 a.m. to 16:30 p.m. All subjects had their own breakfast as usual at least 3 hours before starting the testing session. Subjects chose their lunch from a menu, which was served at exactly 12:30 p.m., and was finished within 20 minutes. The served lunch meals were between 2500 and 3300 kJ. Water was usually available *ad libitum*. Every 15 min subjects donated stimulated saliva, rich in parotid saliva, by chewing on a 25-cm2 piece of parafilm (Parafilm® M) for up to 1 min and spitting into a 12 ml polystyrene tube (Greiner Bio-One, Kremsmünster, Austria). The saliva was immediately placed into a freezer at - 40°C. Also every 15 min subjects were asked to rate "how satiated are you", "how is your desire to eat", "how full are you", and "how hungry are you", using 100-mm non-structured visual analogue line scales (VAS) all on one page and anchored at their ends by the descriptors "very little" and "very much".

SELDI-TOF-MS analysis was performed when testing of subjects was finished and all samples were collected. The SELDI-TOF-MS technology (Ciphergen Biosystems, Fremont, CA, USA) consisted of three major components: the ProteinChip array, a mass spectrometer, and the data analysis software. All common chemicals and reagents were of analytical grade and were obtained from Merck (Darmstadt, Germany), Sigma Aldrich (St Louis. MI), and ACROS Organics (Geel, Belgium), unless stated otherwise. Protein chips, buffers, 3,5-dimethoxy-4-hydroxycinnamic acid, and calibrants were purchased from Ciphergen Biosystems (Ciphergen Biosystems, Fremont, CA). Ammonium acetate was purchased from ICN Biomedicals (Aurora, OH). In the present study, IMAC ProteinChip arrays were used and spots were first loaded with 50 µl 0.1 M copper sulphate by vigorous shaking at room temperature for 10 min. After a short wash with water the chip surface was neutralised using 150 µl sodium acetate buffer (pH 4.0) followed by a short wash with water and preincubation with binding buffer (0.1 M Tris-HCl, pH 7.4, containing 0.1 % Triton X-100, and 100 mM or 500 mM NaCl). Saliva samples were thawed, centrifuged for 2 min at 1000 g at 4°C, and stored on ice. Aliquots were taken from supernatants of the centrifuged saliva, and diluted 1 to 100 in the binding buffer and applied, at random and in duplicate, to the IMAC-Cu chip and incubated for 1 hour shaking on a mixer. Spots were then washed six times with 150 µl of binding buffer for 10 minutes (three times with and without 0.1 % Triton X-100). Before application of the matrix another short wash with water was performed with HPLC-grade water and air-dried for 10 minutes. Sinapinic acid (SPA; (Ciphergen Biosystems, Fremont, CA) was prepared in 50% acetonitrile/0.1 % trifluoroacetic acid according to the manufacturer's instructions. Matrix was applied twice (0.8 µl each time and 1 minute apart), and allowed to air dry again prior to reading on a ProteinChip Reader IIC instrument (Ciphergen Biosystems, Fremont, CA). The following settings were used: detector sensitivity 9; detector voltage 2900; positions 20 to 80 were read with an increment of 10 (resulting in 7 different sampling positions); 50 laser shots were collected on each position (total shots collected and averaged: 350/sample); two warming shots were fired at each position, which were not included in the collection; lag time focus of 241 ns. Laser intensity was optimised for each chip type. Calibration was done with a mixture of proteins with masses ranging from 7 to 30 kDa. After baseline subtraction, peak labelling was performed (CiphergenExpress Software; version 3.0) for peaks with a signal-to-noise (S/N) ratio of≥5 in the m/z range from 1.0 kDa to 35 kDa, and then normalized by total ion current. One hundred ninety peaks common to all spectra were selected and compared with regard to their peak intensity by calculating the average of the duplicate measures.
Mass values obtained from the analyses were searched against Swiss-Prot protein database using the TagIdent tool of ExPASy (27)
(http://us.expasy.org/tools/tagident) with restriction to human classification, pI setting at 5.0 ± 10, and mass tolerance of 10 Da.
Data of all four satiety ratings are expressed as the mean ± SEM. A first correlation analysis was done between BMI, and session means of individual masses (m/z values) obtained from the SELDI-TOF-MS spectra. Further 'across-time' analysis correlated the average values of the 18 subjects at each of the 20 time points of the entire 4¾-hour session with each of the four different sensory ratings on 100-mm VAS and masses (m/z values) by SELDI-TOF-MS. For statistical significance, analysis of variance (ANOVA) was used, and differences with P values of 0.05 or less were considered to be statistically significant. Data were analysed by using Statistica (StatSoft). The resulting SELDI-TOF-MS data set of peak intensities was converted and loaded into a multivariate analysis package (GenStat). Principal components analysis (PCA) was performed to explore the proteomic data sets.

### EXAMPLE 2 - Salivary peptide discovery of satiety in human subjects with SELDI-TOF-MS

The study was aimed at gaining insight into the human saliva proteome composition and its changes over time in response to food consumption. Hereto, within a test session of 4¾ hours, 18 subjects, 9 men and 9 women, were exposed to a lunch-induced hunger-satiety shift, while collecting whole saliva every 15 min. Saliva was quantitatively analyzed for peptide and protein masses by SELDI-TOF-MS, and relative mass intensities of these spectra were related to BMI and subjectively rated scores for satiety across time. This allowed quantification and identification of various expressed peptides as salivary peptides associated with food consumption, perceived satiety, and body mass.
Using IMAC-Cu protein chips in the SELDI-TOF-MS analyses, a total number of 190 peaks in human saliva (from all 18 subjects and 20 time points) were detected with masses between 1293 to 33171 Dalton (130 from 1-10 kDa, 46 from 10-20 kDa, and 14 from 20-40 kDa). The majority of peaks was between 1200 and 15000, demonstrating that the sensitivity of this approach and protein chip type appears to be high mainly for peptides between 1200 and 15000.

**Table 2. Experimental masses (m/z) detected in human saliva by SELDI-TOF-MS on IMAC ProteinChips. Peak intensity represents ion current (coefficient of variation) averaged over the 18 subj ects**

| **peak** | **m/z** | **intensity** | **peak** | **m/z** | **intensity** | **peak** | **m/z** | **intensity** |
|---|---|---|---|---|---|---|---|---|
| A1 | 1293 | 9.3(0.46) | H2 | 4717 | 1.8 (1.39) | 02 | 9734 | 1.1 (0.91) |
| A2 | 1361 | 3.5 (1.20) | H3 | 4786 | 0.9 (2.44) | 03 | 9846 | 0.5 (0.80) |
| A3 | 1396 | 3.9 (1.43) | H4 | 4806 | 6.7(0.81) | 04 | 9935 | 0.3 (0.67) |
| A4 | 1434 | 19.0(0.37) | H5 | 4820 | 2.1 (0.90) | 05 | 10240 | 0.7 (1.00) |
| A5 | 1439 | 4.1 (0.58) | H6 | 4836 | 5.5 (0.75) | 06 | 10338 | 3.8 (0.71) |
| A6 | 1479 | 5.5 (1.12) | H7 | 4899 | 3.4 (0.59) | 07 | 10424 | 2.0(0.65) |
| A7 | 1567 | 8.0 (0.74) | H8 | 4919 | 19.4 (0.45) | 08 | 10648 | 0.7 (0.86) |
| A8 | 1593 | 0.8(3.13) | H9 | 4943 | 5.9 (0.85) | 09 | 10727 | 0.9 (0.89) |
| A9 | 1659 | 2.9 (1.45) | 11 | 4964 | 2.4 (1.00) | P1 | 10814 | 9.2(0.60) |
| B1 | 1693 | 10.6(0.54) | 12 | 5005 | 6.1 (0.80) | P2 | 10837 | 16.5 (0.61) |
| B2 | 1722 | 6.8 (0.15) | 13 | 5075 | 2.7 (1.41) | P3 | 11017 | 2.7 (0.30) |
| B3 | 1737 | 8.1 (0.89) | 14 | 5121 | 5.8(0.74) | P4 | 11133 | 3.2(0.59) |
| B4 | 1754 | 1.6(0.63) | 15 | 5141 | 2.4 (0.71) | P5 | 11189 | 2.7 (0.48) |
| B5 | 1902 | 3.9(0.95) | 16 | 5148 | 1.6(0.81) | P6 | 11200 | 4.5(0.18) |
| B6 | 1951 | 2.6 (1.04) | 17 | 5155 | 3.2(0.63) | P7 | 11468 | 1.1 (1.00) |
| B7 | 2028 | 1.1 (1.55) | 18 | 5269 | 2.0(1.35) | P8 | 11498 | 2.8 (1.21) |
| B8 | 2070 | 4.5 (1.07) | 19 | 5303 | 1.3 (1.54) | P9 | 11580 | 0.4 (1.00) |
| B9 | 2117 | 13.4 (0.62) | J1 | 5360 | 1.0 (0.60) | Q1 | 11704 | 3.5 (0.46) |
| C1 | 2234 | 4.9 (0.78) | J2 | 5377 | 2.0(1.45) | Q2 | 11716 | 6.9 (0.58) |
| C2 | 2255 | 2.6 (1.35) | J3 | 5415 | 6.7(0.88) | Q3 | 11886 | 1.7(0.47) |
| C3 | 2274 | 2.3 (0.99) | J4 | 5518 | 1.5 (0.93) | Q4 | 11907 | 1.0 (0.60) |
| C4 | 2316 | 2.6 (1.12) | J5 | 5572 | 1.7(0.47) | Q5 | 12130 | 0.3(0.67) |
| C5 | 2387 | 4.2(0.74) | J6 | 5681 | 2.1 (1.05) | Q6 | 12502 | 0.6(0.67) |
| C6 | 2462 | 5.1 (0.84) | J7 | 5727 | 1.6(0.50) | Q7 | 12588 | 2.9 (0.59) |
| C7 | 2480 | 2.5 (1.44) | J8 | 5852 | 1.6(0.44) | Q8 | 12641 | 8.8 (0.53) |
| C8 | 2503 | 4.8 (0.63) | J9 | 6223 | 4.7 (0.91) | Q9 | 12673 | 5.6(0.68) |
| C9 | 2553 | 1.0 (2.20) | K1 | 6289 | 6.5 (0.43) | R1 | 12865 | 1.0 (0.50) |
| D1 | 2587 | 2.2 (1.27) | K2 | 6313 | 11.6(0.69) | R2 | 13160 | 0.9(0.67) |
| D2 | 2619 | 27.2 (0.30) | K3 | 6380 | 3.4 (0.53) | R3 | 13337 | 1.2 (0.33) |
| D3 | 2697 | 1.0(1.50) | K4 | 6623 | 8.3 (0.87) | R4 | 13440 | 4.0 (0.58) |
| D4 | 2711 | 1.0(1.10) | K5 | 6636 | 17.7 (1.07) | R5 | 13449 | 2.1 (0.52) |
| D5 | 2746 | 12.3 (0.76) | K6 | 6721 | 1.0 (0.70) | R6 | 13530 | 2.0 (0.40) |
| D6 | 2780 | 1.8 (2.06) | K7 | 6777 | 4.6 (0.87) | R7 | 13544 | 3.5 (0.60) |
| D7 | 2822 | 3.1 (1.00) | K8 | 6818 | 1.8(0.67) | R8 | 13636 | 1.9(0.63) |
| D8 | 2874 | 8.5 (1.02) | K9 | 6884 | 1.7 (0.94) | R9 | 13661 | 3.2 (0.53) |
| D9 | 2908 | 0.5 (1.60) | L1 | 7137 | 6.9 (0.58) | S1 | 13783 | 2.2(0.86) |
| E1 | 2967 | 2.6 (1.42) | L2 | 7146 | 3.9(0.49) | S2 | 13810 | 3.7(0.92) |
| E2 | 3010 | 22.8 (0.36) | L3 | 7212 | 0.7 (2.00) | S3 | 14131 | 0.4 (0.75) |
| E3 | 3021 | 50.8 (0.39) | L4 | 7253 | 0.7(0.57) | S4 | 14282 | 5.4 (0.52) |
| E4 | 3033 | 2.6 (1.35) | L5 | 7315 | 7.2(0.68) | S5 | 14495 | 1.4 (0.57) |
| E5 | 3157 | 22.2 (0.44) | L6 | 7338 | 4.1 (0.63) | S6 | 14641 | 14.4 (0.60) |
| E6 | 3214 | 1.2 (1.42) | L7 | 7447 | 1.8 (0.44) | S7 | 14657 | 7.5 (0.59) |
| E7 | 3285 | 13.2(0.56) | L8 | 7537 | 0.6(0.50) | S8 | 14849 | 1.9 (0.68) |
| E8 | 3370 | 24.4 (0.42) | L9 | 7647 | 1.9 (0.58) | S9 | 14861 | 3.5 (0.69) |
| E9 | 3438 | 29.5(0.27) | M1 | 7701 | 1.8(0.72) | T1 | 15491 | 13.6 (0.41) |
| F1 | 3480 | 16.8 (0.65) | M2 | 7744 | 8.3 (0.47) | T2 | 15510 | 27.9 (0.46) |
| F2 | 3502 | 2.3 (1.74) | M3 | 7757 | 18.2(0.51) | T3 | 15674 | 3.3(0.42) |
| F3 | 3592 | 2.3 (0.70) | M4 | 7837 | 2.1 (0.33) | T4 | 17574 | 0.6 (1.67) |
| F4 | 3643 | 4.5 (0.76) | M5 | 8098 | 5.1 (1.06) | T5 | 17613 | 1.9 (1.52) |
| F5 | 3661 | 9.1 (0.84) | M6 | 8122 | 4.0 (0.98) | T6 | 20437 | 0.3 (0.68) |
| F6 | 3915 | 3.6 (0.50) | M7 | 8228 | 0.8 (1.13) | T7 | 23741 | 0.3 (0.66) |
| F7 | 3994 | 6.1 (1.15) | M8 | 8280 | 6.2 (1.00) | T8 | 23949 | 0.2(0.49) |
| F8 | 4051 | 3.4 (1.06) | M9 | 8334 | 0.8 (0.75) | T9 | 25469 | 0.2(0.50) |
| F9 | 4086 | 0.9 (1.78) | N1 | 8414 | 1.3(0.77) | U1 | 26743 | 0.2(0.52) |
| G1 | 4129 | 9.4 (0.46) | N2 | 8549 | 0.9 (0.78) | U2 | 28073 | 0.2(0.49) |
| G2 | 4183 | 6.8 (0.59) | N3 | 8725 | 1.0 (1.10) | U3 | 29040 | 0.2 (0.50) |
| G3 | 4370 | 14.0(0.29) | N4 | 8922 | 1.6 (1.31) | U4 | 29110 | 0.4 (0.49) |
| G4 | 4414 | 1.8 (0.83) | N5 | 9028 | 1.5 (1.60) | U5 | 29740 | 0.2(0.49) |
| G5 | 4501 | 3.7 (1.19) | N6 | 9123 | 0.6 (0.83) | U6 | 29956 | 0.4 (0.48) |
| G6 | 4519 | 5.0(0.56) | N7 | 9197 | 1.3 (0.62) | U7 | 30081 | 0.2(0.50) |
| G7 | 4564 | 2.5 (0.76) | N8 | 9281 | 0.6 (1.00) | U8 | 30941 | 0.6(0.83) |
| G8 | 4590 | 2.0(1.85) | N9 | 9507 | 1.0(0.90) | U9 | 30991 | 0.9 (0.67) |
| G9 | 4614 | 1.2(0.58) | O1 | 9645 | 0.5 (0.80) | V1 | 33171 | 0.2(0.49) |
| H1 | 4688 | 2.4 (0.92) | | | | | | |

**Table 3. Experimental masses (m/z) by SELDI-TOF-MS analysis (IMAC ProteinChip) on human saliva with correlation coefficients (r) in relation to VAS ratings for satiety, desire to eat, fullness, and hunger across 20 time points averaged over 18 subjects. M/z-values with highly significant correlations to satiety ratings (0.70<|r|<0.95) are in bold**

| **peak** | ***m*/*z*** | ***r* satiety** | ***r* desire** | ***r* fullness** | ***r* hunger** | **peak** | **m/z** | ***r* satiety** | ***r* desire** | ***r* fullness** | ***r* hunger** |
|---|---|---|---|---|---|---|---|---|---|---|---|
| A1 | 1293 | -0.52^{a} | 0.55^{a} | -0.54^{a} | 0.51^{a} | K9 | 6884 | -0.68^{c} | 0.57^{b} | -0.71^{c} | 0.57^{b} |
| **A2** | **1361** | **-0.73^{c}** | **0.73^{c}** | **-0.71^{c}** | **0.67^{b}** | L3 | 7212 | -0.68^{c} | 0.63^{b} | -0.70^{c} | 0.70^{c} |
| A4 | 1439 | -0.67^{b} | 0.71^{c} | -0.67^{b} | 0.71^{c} | M1 | 7701 | 0.53^{a} | -0.45^{a} | 0.55^{a} | -0.43 |
| A8 | 1593 | -0.49^{a} | 0.38 | -0.56^{b} | 0.45^{a} | M2 | 7744 | 0.64^{b} | -0.55^{a} | 0.66^{b} | -0.52^{a} |
| B1 | 1693 | -0.59^{b} | 0.51^{a} | -0.62^{b} | 0.48^{a} | M3 | 7757 | 0.53^{a} | -0.48^{a} | 0.54^{a} | -0.40 |
| B2 | 1722 | -0.65^{b} | 0.60^{b} | -0.64^{b} | 0.62^{b} | **M5** | **8098** | **-0.73^{c}** | **0.66^{b}** | **-0.75^{c}** | **0.68^{c}** |
| **B3** | **1737** | **-0.76^{c}** | **0.75^{c}** | **-0.76^{c}** | **0.69^{c}** | **M6** | **8122** | **-0.80^{c}** | **0.74^{c}** | **-0.83^{c}** | **0.77^{c}** |
| **B6** | **1951** | **-0.82^{c}** | **0.76^{c}** | **-0.84^{c}** | **0.78^{c}** | M7 | 8228 | -0.61^{b} | 0.50^{a} | -0.66^{b} | 0.53^{a} |
| C6 | 2462 | 0.63^{b} | -0.54^{a} | 0.66^{b} | -0.52^{a} | M8 | 8280 | -0.60^{b} | 0.58^{b} | -0.59^{b} | 0.55^{a} |
| D5 | 2746 | 0.61^{b} | -0.59^{b} | 0.59^{b} | -0.68^{c} | **M9** | **8334** | **-0.75^{c}** | **0.69^{c}** | **-0.80^{c}** | **0.73^{c}** |
| **D6** | **2780** | **-0.83^{c}** | **0.84^{c}** | **-0.83^{c}** | **0.85^{c}** | N1 | 8414 | 0.47^{a} | -0.47^{a} | 0.45^{a} | -0.51^{a} |
| **D8** | **2874** | **-0.81^{c}** | **0.78^{c}** | **-0.79^{c}** | **0.70^{c}** | **N3** | **8725** | **-0.78^{c}** | **0.73^{c}** | **-0.83^{c}** | **0.74^{c}** |
| E3 | 3021 | -0.64^{b} | 0.61^{b} | -0.61^{b} | 0.59^{b} | **N4** | **8922** | **-0.70^{c}** | **0.62^{b}** | **-0.72^{c}** | **0.68^{c}** |
| E4 | 3033 | 0.62^{b} | -0.54^{a} | 0.64^{b} | -0.50^{a} | **N6** | **9123** | **-0.92^{c}** | **0.88^{c}** | **-0.91^{c}** | **0.82^{c}** |
| E5 | 3157 | 0.53^{a} | -0.45^{a} | 0.57^{b} | -0.48^{a} | N7 | 9197 | -0.54^{a} | 0.48^{a} | -0.50^{a} | 0.39 |
| E6 | 3214 | -0.57^{b} | 0.50^{a} | -0.59^{b} | 0.49^{a} | **N8** | **9281** | **-0.76^{c}** | **0.69^{c}** | **-0.77^{c}** | **0.71^{c}** |
| E7 | 3285 | 0.46^{a} | -0.41 | 0.47^{a} | -0.45^{a} | **N9** | **9507** | **-0.90^{c}** | **0.87^{c}** | **-0.92^{c}** | **0.86^{c}** |
| **E8** | **3370** | **-0.72^{c}** | **0.64^{b}** | **-0.73^{c}** | **0.64^{b}** | **O1** | **9645** | **-0.71^{c}** | **0.66^{b}** | **-0.70^{c}** | **0.58^{b}** |
| **E9** | **3438** | **-0.80^{c}** | **0.75^{c}** | **-0.78^{c}** | **0.74^{c}** | O2 | 9734 | -0.67^{b} | 0.64^{b} | -0.70^{c} | 0.58^{b} |
| **F1** | **3480** | **-0.70^{c}** | **0.64^{b}** | **-0.72^{c}** | **0.63^{b}** | O3 | 9846 | 0.54^{a} | -0.48^{a} | 0.56^{a} | -0.48^{a} |
| F7 | 3994 | 0.67^{b} | -0.61^{b} | 0.65^{b} | -0.61^{b} | 04 | 9935 | 0.56^{b} | -0.49^{a} | 0.58^{b} | -0.48^{a} |
| F8 | 4051 | 0.46^{a} | -0.39 | 0.50^{a} | -0.36 | O8 | 10648 | -0.64^{b} | 0.54^{a} | -0.64^{b} | 0.46^{a} |
| F9 | 4086 | 0.54^{a} | -0.47^{a} | 0.56^{a} | -0.45^{a} | P1 | 10814 | -0.48^{a} | 0.38 | -0.53^{a} | 0.37 |
| G1 | 4129 | -0.50^{a} | 0.45^{a} | -0.54^{a} | 0.43 | P2 | 10837 | -0.48^{a} | 0.40 | -0.51^{a} | 0.36 |
| G2 | 4183 | -0.45^{a} | 0.46^{a} | -0.43 | 0.52^{a} | P3 | 11017 | -0.54^{a} | 0.45^{a} | -0.58^{b} | 0.44 |
| G4 | 4414 | -0.55^{a} | 0.68^{c} | -0.58^{b} | 0.70^{c} | Q3 | 11886 | 0.54^{a} | -0.50^{a} | 0.54^{a} | -0.51^{a} |
| G5 | 4501 | 0.46^{a} | -0.50^{a} | 0.49^{a} | -0.56^{a} | Q4 | 11907 | 0.52^{a} | -0.42 | 0.50^{a} | -0.44 |
| G9 | 4614 | -0.50^{a} | 0.50^{a} | -0.56^{a} | 0.55^{a} | Q5 | 12130 | -0.59^{b} | 0.53^{a} | -0.60^{b} | 0.43 |
| **H1** | **4688** | **0.82^{c}** | **-0.74^{c}** | **0.85^{c}** | **-0.70^{c}** | Q6 | 12502 | -0.47^{a} | 0.45^{a} | -0.48^{a} | 0.44 |
| H5 | 4820 | 0.53^{a} | -0.49^{a} | 0.57^{b} | -0.52^{a} | Q7 | 12588 | -0.53^{a} | 0.50^{a} | -0.60^{b} | 0.46^{a} |
| H6 | 4836 | 0.65^{b} | -0.57^{b} | 0.58^{c} | -0.57^{b} | Q8 | 12641 | -0.56^{a} | 0.49^{a} | -0.58^{b} | 0.43 |
| **H7** | **4899** | **0.72^{c}** | **-0.64^{b}** | **0.74^{c}** | **-0.63^{b}** | Q9 | 12673 | -0.56^{b} | 0.50^{a} | -0.58^{b} | 0.45^{a} |
| **H8** | **4919** | **0.72^{c}** | **-0.63^{b}** | **0.74^{c}** | **-0.59^{b}** | R1 | 12865 | -0.46^{a} | 0.38 | -0.50^{a} | 0.34 |
| **H9** | **4943** | **0.78^{c}** | **-0.70^{c}** | **0.80^{c}** | **-0.70^{c}** | R8 | 13636 | -0.61^{b} | 0.53^{a} | -0.65^{b} | 0.47^{a} |
| **I1** | **4964** | **0.72^{c}** | **-0.64^{b}** | **0.75^{c}** | **-0.67^{b}** | S1 | 13783 | -0.67^{b} | 0.59^{b} | -0.71^{c} | 0.60^{b} |
| I**2** | **5005** | **0.70^{c}** | **-0.59^{b}** | **0.71^{c}** | **-0.57^{b}** | S2 | 13810 | -0.44^{a} | 0.35 | -0.46^{a} | 0.35 |
| I3 | 5075 | 0.68^{b} | -0.59^{b} | 0.69^{c} | -0.57^{b} | T1 | 15491 | 0.66^{b} | -0.56^{a} | 0.67^{b} | -0.53^{a} |
| **I4** | **5121** | **0.74^{c}** | **-0.65^{b}** | **0.77^{c}** | **-0.64^{b}** | T2 | 15510 | 0.64^{b} | -0.57^{b} | 0.64^{b} | -0.50^{a} |
| I5 | 5141 | 0.62^{b} | -0.54^{a} | 0.63^{b} | -0.53^{a} | T3 | 15674 | 0.63^{b} | -0.53^{a} | 0.65^{b} | -0.49^{a} |
| I9 | 5303 | -0.57^{b} | 0.49^{a} | 0.28 | 0.50^{a} | T4 | 17574 | 0.64^{b} | -0.59^{b} | 0.67^{b} | -0.55^{a} |
| **J6** | **5681** | **-0.71^{c}** | **0.70^{c}** | **-0.69^{c}** | **0.72^{c}** | **T5** | **17613** | **0.77^{c}** | **-0.70^{c}** | **0.79^{c}** | **-0.69^{c}** |
| J8 | 5852 | -0.61^{b} | 0.52^{a} | -0.68^{b} | 0.53^{a} | T6 | 20437 | 0.53^{a} | -0.45^{a} | 0.55^{a} | -0.42 |
| J9 | 6223 | -0.68^{b} | 0.60^{b} | -0.72^{c} | 0.67^{b} | U5 | 29740 | 0.65^{b} | -0.60^{b} | 0.67^{b} | -0.58^{b} |
| K1 | 6289 | -0.57^{b} | 0.53^{a} | -0.62^{b} | 0.55^{a} | U6 | 29956 | 0.51^{a} | -0.53^{a} | 0.51^{a} | -0.53^{a} |
| K2 | 6313 | -0.48^{a} | 0.44 | -0.52^{a} | 0.50^{a} | **U7** | **30081** | **0.71^{c}** | **-0.66^{b}** | **0.71^{c}** | **-0.68^{c}** |
| K4 | 6623 | -0.58^{b} | 0.51^{a} | -0.62^{b} | 0.59^{b} | **U8** | **30941** | **0.73^{c}** | **-0.65^{b}** | **0.74^{c}** | **-0.63^{b}** |
| K5 | 6636 | -0.49^{a} | 0.45^{a} | -0.53^{a} | 0.55^{a} | **U9** | **30991** | **0.70^{c}** | **-0.64^{b}** | **0.69^{c}** | **-0.60^{b}** |
| K7 | 6777 | -0.50^{a} | 0.47^{a} | -0.50^{a} | 0.51^{a} | V1 | 33171 | 0.53^{a} | -0.48^{a} | 0.51^{a} | -0.43 |
| K8 | 6818 | -0.67^{b} | 0.59^{b} | -0.73^{c} | 0.63^{b} | | | | | | |

| | | | | | | | | | | | |
|---|---|---|---|---|---|---|---|---|---|---|---|
| a: * *p* < 0.05; b: * *p* < 0.01; c: * *p* < 0.001 | | | | | | | | | | | |

From the 190 peaks, 29 had a strong correlation (0.70<r<0.95) with all four subjective satiety ratings across time during the hunger-satiety test session (table 5). The mean intensities of 7 peaks were significantly correlated to BMI, table 4.

**Table 4. Significant correlations (r = correlation coefficient) between BMI and the session mean of peak intensity of experimental masses (m/z) detected in human saliva by SELDI-TOF-MS on IMAC ProteinChips including 20 repeated observations in time in 18 subjects. M/z 8122 (M6) (bold) also highly related negatively to satiety. M/z-values 6721 (K6), 13,160 (R2), and 23,949 (T8) (italic) were not significantly related to satiety ratings.**

| **peak** | ***m*/*z*** | ***r* BMI** | **peak** | ***m*/*z*** | ***r* BMI** |
|---|---|---|---|---|---|
| G5 | 4501 | -0.49 | P3 | 11017 | 0.66 |
| *K6* | *6721* | *0.58* | *R2* | *13160* | *0.58* |
| **M6** | **8122** | **0.53** | *T8* | *23949* | *0.55* |
| P1 | 10814 | 0.56 | | | |

| | | | | | |
|---|---|---|---|---|---|
| *p* < 0.05 | | | | | |

### EXAMPLE 3 - Identification of ghrelin, glucagon and peptide YY in saliva

The 30 peptides and proteins from the present ProteinChip analyses, which strongly correlated with the four satiety ratings, were searched in the SwissProt protein database using TagIdent (ExPASy). As shown in Table 5, several peptides and proteins were reported to have a similar molecular weight to some of the *m*/*z* values found. Some of the molecular weights are most likely metabolically-related substances, in particular gastrointestinal and pancreatic peptide hormones; e.g. 2780 (D6) (insulin-like 3 A chain or insulin-like peptide A-chain]), 3370 (E8) (ghrelin-28 [octynoylated]), 3438 (E9) (insulin B chain), 3480 (F1) (glucagon), and 5005 (I2) (somatomedin B). Furthermore, as illustrated in Table 4, there are m/z values that possibly corresponded to specific salivary peptides, i.e. 3370 (E8) (neutrophil defensin-2), 3438 (E9) (histatin-2), and 4964 (I1) (beta-defensin 126). The m/z 8122 (M6), related both to satiety and BMI, was not yet identified.

**Table 5. Experimental masses (m/z) by SELDI-TOF-MS analysis (IMAC-Cu ProteinChip) on human saliva compared to identified peptides and proteins with similar molecular weight from SwissProt database (TagIdent tool of ExPASy) with restriction to human classification and mass tolerance of 10 Da**

| **peak** | ***m*/*z*** | **peptide/protein description** | accession number (- SEQ ID No: ) | **molecular weight (Da)** |
|---|---|---|---|---|
| A2 | 1361 | neuropeptide SF | O15130 - 1 | 1368.56 |
| B3 | 1737 | kisspeptin-14 | Q15726 - 2 | 1742.91 |
| B6 | 1951 | unknown | | |
| D6 | 2780 | insulin-like 3A chain | P51460 - 3 | 2777.20 |
| | | early placenta insulin-like peptide A chain | Q14641 - 4 | 2789.17 |
| D8 | 2874 | P-beta | Q9H300 - 5 | 2876.23 |
| E8 | 3370 | ghrelin-28 (O-octanoylated) | Q9UBU3 - 6 | 3371.3 |
| | | neutrophil defensin-2 | P59665 - 7 | 3377.01 |
| | | hypothetical protein C21 orf89 | P59042 - 8 | 3376.80 |
| E9 | 3438 | insulin B chain | P01308 - 9 | 3429.96 |
| | | histatin-2 | P15515 - 10 | 3444.69 |
| F1 | 3480 | glucagon | P01275 - 11 | 3482.79 |
| | | C-flanking peptide of NPY | P01303 - 12 | 3474.91 |
| F8 | 4050 | Peptide YY | - 13 | |
| H1 | 4688 | BAX protein, cytoplasmic isoform γ | Q07815 - 14 | 4678.22 |
| | | urocortin | P55089 - 15 | 4697.29 |
| H7 | 4899 | Cyt c oxidase PP VIII-liver/heart, mit. precursor | P10176 - 16 | 4893.78 |
| | | thymosin beta-10 | P63313 - 17 | 4894.48 |
| H8 | 4919 | thymosin beta-4 | P62328 - 18 | 4921.46 |
| H9 | 4943 | unknown | | |
| I1 | 4964 | beta-defensin 126 | Q9BYW3 - 19 | 4957.89 |
| 12 | 5005 | somatomedin B | P04004 - 20 | 5011.54 |
| 14 | 5121 | unknown | | |
| J6 | 5681 | granulin-5 | P28799 -21 | 5671.30 |
| M5 | 8098 | small inducible cytokine A22 | 000626 - 22 | 8090.47 |
| | | glicentin | P01275 - 23 | 8100.74 |
| | | IL-8(9-77) | P10145-24 | 8099.44 |
| | | neutrophil-activating peptide 2 (74) | P02775 - 25 | 8106.44 |
| M6 | 8122 | unknown | | |
| M9 | 8334 | unknown | | |
| N3 | 8725 | basic nuclear protein HPS2 | P04554 - 26 | 8723.00 |
| | | CSL-type zinc finger-containing protein 1 | Q9H4G8 - 27 | 8720.7 |
| N4 | 8922 | apolipoprotein C-II | P02655 - 28 | 8914.92 |
| | | small inducible cytokine A8 | P80075 - 29 | 8914.44 |
| | | interleukin-8 | P10145 - 30 | 8922.45 |
| | | activation peptide | P00747 - 31 | 8922.12 |
| | | secreted Ly-6/uPAR-related protein 1 | P55000 - 32 | 8924.23 |
| | | ubiquitin-fold modifier 1 | P61960 - 33 | 8927.36 |
| N6 | 9123 | keratin-associated protein 19-6 | Q3LI70 - 34 | 9125.27 |
| | | U6 snRNA-associated Sm-like protein LSm6 | P62312 - 35 | 9127.57 |
| | | Saposin D | P07602 - 36 | 9126.58 |
| N8 | 9281 | Down syndrome critical regulatory protein 10 | P59022 - 37 | 9285.92 |
| | | keratin-associated protein 7-1 | Q8IUC3 - 38 | 9288.31 |
| | | NADH dehydrogenase 1 α subcomplex subunit 3 | O95167 - 39 | 9278.85 |
| N9 | 9507 | ATPase inhibitor | Q9UII2 - 40 | 9516.56 |
| | | hypothetical protein C9orf27 | Q9P2X8 - 41 | 9514.92 |
| | | keratin-associated protein 17-1 | Q9BYP8 - 42 | 9503.83 |
| | | Late cornified envelope protein 3E | Q5T5B0 - 43 | 9506.64 |
| | | stannin | 075324 - 44 | 9497.10 |
| O1 | 9645 | retinal rod rhodopsin-sensitive cGMP PDE γ- | P18545 - 45 | 9643.13 |
| | | subunit | | |
| T5 | 17613 | COMM domain-containing protein 6 | Q7Z4G1 - 46 | 9638.10 |
| | | thioredoxin domain-containing protein C9orf121 | Q5VZ03 - 47 | 17614.32 |
| | | HERV-K_3q21.2 provirus ancestral Gag | P62688 - 48 | 17611.30 |
| | | polyprotein | | |
| | | 60S ribosomal protein L29 | P47914 - 49 | 17620.88 |
| U7 | 30081 | protein SYS1 homolog | Q8N2H4 - 50 | 17614.90 |
| | | zinc finger HIT domain-containing protein 3 | Q15649 - 51 | 17607.28 |
| | | ADP-ribosyl cyclase 2 | Q10588 - 52 | 30082.05 |
| U8 | 30941 | methyltransferase-like protein 6 | Q8TCB7 - 53 | 30077.21 |
| | | THAP domain-containing protein 8 | Q8NA92 - 54 | 30081.65 |
| | | ubiquinone biosynthesis protein COQ9 | 075208 - 55 | 30940.50 |
| | | homeobox protein MSX-1 | P28360 - 56 | 30939.64 |
| | | SPRY domain-containing SOCS box protein 1 | Q96BD6 - 57 | 30941.55 |
| | | tissue-type plasmonigen activator chain A | P00750 - 58 | 30938.54 |
| U9 | 30991 | testis expressed sequence 264 protein | Q9Y619 - 59 | 30946.53 |
| | | transcription cofactor vestigial-like protein 4 | Q14135 - 60 | 30947.54 |
| | | NG,NG-dimethylarginine methylaminohydrolase 1 | 094760 - 61 | 30990.58 |
| | | elongation factor 1-δ | P29692 - 62 | 30990.64 |

PYY is a naturally occurring human hormone produced by produced in the small intestine and colon, in particular by specialized endocrine cells (L-cells) in the gut in proportion. The GI peptide hormone YY belongs to the pancreatic polypeptide (PP) family along with PP and neuropeptide Y (NPY). These peptides mediate their effects through the NPY receptors of which there are several subtypes (Y1, Y2, Y4, and Y5). PYY exists in two endogenous forms: PYY(1-36) and PYY(3-36). The latter is produced by the action of the enzyme dipeptidyl peptidase-IV (DPP-IV). PYY(1-36) binds to and activates at least three Y receptor subtypes (Y1, Y2, and Y5), whereas PYY(3-36) is more selective for Y2 receptor (Y2R). The hypothalamic arcuate nucleus, a key brain area regulating appetite, has access to nutrients and hormones within the peripheral circulation. NPY neurons within the arcuate nucleus express the Y2R. In response to food ingestion plasma PYY(3-36) concentrations rise.
Ghrelin is a hormone produced by P/D1 cells lining the fundus of the human stomach that stimulate appetite. Ghrelin levels increase before meals and decrease after meals. It is considered the counterpart of the hormone leptin, produced by adipose tissue, which reduces appetite when present at higher levels. Obestatin is a hormone that was found, in late 2005, to decrease appetite. Both obestatin and ghrelin are encoded by the same gene; the gene's product breaks apart to yield the two peptide hormones. Receptors for ghrelin are expressed by neurons in the arcuate nucleus and the ventromedial hypothalamus. The ghrelin receptor is a G-protein coupled membrane receptor, formerly known as the GHS receptor (growth hormone secretagogue receptor). A recent study found that gastric bypass surgery not only reduces the gut's capacity for food, but also dramatically lowers ghrelin levels (28). Ghrelin is also made by a small population of neurons in the arcuate nucleus and plays a role in neurotrophy, particularly in the hippocampus, and is essential for cognitive adaption to changing environments and the process of learning. Ghrelin exists in the body as an inactive and an active octanoylated form.
Glucagon is a 29-amino acid polypeptide acting as an important hormone in carbohydrate metabolism and facilitates the body to maintain proper blood sugar levels. The polypeptide has a molecular weight of 3485 daltons. The hormone is synthesized and secreted from alpha cells (α-cells) of the islet of Langerhans, which are located in the endocrine portion of the pancreas. Glucagon helps maintain the level of glucose in the blood by binding to glucagon receptors on hepatocytes, causing the liver to release glucose through glycogenolysis. As these stores become depleted, glucagon stimulates the liver to synthesize glucose by gluconeogenesis, that is released into the bloodstream. Both of these mechanisms lead to glucose release by the liver, preventing the development of hypoglycemia.

**Table 6. Amino acid sequences (N to C-termini) of a selection of identified peptides and proteins from SwissProt database (TagIdent tool ofExPASy) with restriction to human classification**

| | |
|---|---|
| Insulin α chain | |
| G IVEQCCTSICSLYQLENYCN (SEQ ID NO : 63) | |
| Insulin β chain | |
| FVNQHLCGSHLVEALYLVCGERGFFYTPKT (SEQ ID NO : 64) | |
| Insulin-like 3A chain | |
| AAATNPARYCCLSGCTQQDLLTLCPY (SEQ ID NO : 65) | |
| Somatostatins | |
| | Somatostatin-14 |
| AGCKNFFWKTFTSC (SEQ ID NO : 66) | |
| | somatostatin-28 |
| SANSNPAMAPRERKAGCKNFFWKTFTSC (SEQ ID NO : 67) | |
| Gastrins | |
| | Gastrin-6 |
| | YGWMDF (SEQ ID NO : 68) |
| | Gastrin-14 |
| | WLEEEEEAYGWMDF (SEQ ID NO : 69) |
| | Gastrin-34 |
| | QLGPQGPPHLVADPSKKQGPWLEEEEEAYGWMDF (SEQ ID NO: 70) |
| | Gastrin-52 |
| | DLELPWLEQQGPASHHRRQLGPQGPPHLVADPSKKQGPWLEEEEEAYG |
| | WMDF (SEQ ID NO : 71) |
| | Gastrin-71 |
| SWKPRSQQPDAPLGTGANRDLELPWLEQQGPASHHRRQLGPQGPPHLVADPS KKQGPWLEEEEEAYGWMDF (SEQ ID NO : 72) | |
| Cholecystokinin (CCK) | |
| | CCK-8 |
| | DYMGWMDF (SEQ ID NO : 73) |
| | CCK-12 |
| | ISDRDYMGWMDF (SEQ ID NO : 74) |
| | CCK-33 |
| | KAPSGRMSIVKNLQNLDPSHRISDRDYMGWMDF (SEQ ID NO : 75) |
| | CCK-39 |
| | YIQQARKAPSGRMSIVKNLQNLDPSHRISDRDYMGWMDF |
| | (SEQ ID NO : 76) |
| | CCK-58 |
| | VSQRTDGESRAHLGALLARYIQQARKAPSGRMSIVKNLQNLDPSHRISDR DYMGWMDF (SEQ ID NO : 77) |
| | Cholecystokinin |
| | QPVPPADPAGSGLQRAEEAPRRQLRVSQRTDGESRAHLGALLARYIQQA RKAPSGRMSIVKNLQNLDPSHRISDRDYMGWMDFGRRSAEEYEYPS (SEQ ID NO : 78) |
| Secretin | |
| HSDGTFTSELSRLREGARLQRLLQGLV (SEQ ID NO : 79) | |
| Motilin | |
| FVPIFTYGELQRMQEKERNKGQ (SEQ ID NO : 80) | |
| vasoactive intestinal peptide (VIP) | |
| HSDAVFTDNYTRLRKQMAVKKYLNSILN (SEQ ID NO : 81) | |
| peptide YY (PYY) 3-36 | |
| IKPEAPGEDASPEELNRYYASLRHYLNLVTRQRY (SEQ ID NO : 82) | |
| pancreatic hormone | |
| APLEPVYPGDNATPEQMAQYAADLRRYINMLTRPRY SEQ ID NO : 83) | |
| pancreatic icosapeptide | |
| HKEDTLAFSEWGSPHAAVPR (SEQ ID NO : 84) | |
| ghrelin-28 | |
| GSS(octanyl)FLSPEHQRVQQRKESKKPPAKLQPR (SEQ ID NO : 85) | |
| Glucagon | |
| HSQGTFTSDYSKYLDSRRAQDFVQWLMNT (SEQ ID NO : 86) | |
| glucagon-like peptide 1 (GLP-1) | |
| HDEFERHAEGTFTSDVSSYLEGQAAKEFIAWLVKGRG (SEQ ID NO : 87) | |
| glucagon-like peptide 2 (GLP-2) | |
| HADGSFSDEMNTILDNLAARDFINWLIQTKITD (SEQ ID NO : 88) | |
| glicentin | |
| RSLQDTEEKSRSFSASQADPLSDPDQMNEDKRHSQGTFTSDYSKYLDSRRAQD FVQWLMNTKRNRNNIA (SEQ ID NO : 89) | |
| gastric inhibitory polypeptide / glucose-dependent insulinotropic polypeptide (GIP) | |
| YAEGTFISDYSIAMDKIHQQDFVNWLLAQKGKKNDWKHNITQ | |
| (SEQ ID NO : 90) | |
| Amylin | |
| KCNTATC ATQRLANFLV HSSNNFGAIL SSTNVGSNTY | |
| (SEQ ID NO : 91) | |
| Neutrophil defensin-2 | |
| CYCRIPACIAGERRYGTCIYQGRLWAFCC (SEQ ID NO : 92) | |
| somatomedin B (insulin-like growth factor) | |
| DQESCKGRCTEGFNVDKKCQCDELCSYYQSCCTDYTAECKPQVT | |
| (SEQ ID NO : 93) | |
| Histatin-2 | |
| RKFHEKHHSHREFPFYGDYGSNYLYDN (SEQ ID NO : 94) | |
| Beta-defensin 126 | |
| NWYVKKCLNDVGICKKKCKPEEMHVKNGWAMCGKQRDCCVPAD | |
| (SEQ ID NO : 95) | |
| Leptin | |
| VPIQKVQDDTKTLIKTIVTRINDISHTQSVSSKQKVTGLDFIPGLHPIL TLSKMDQTLAVYQQILTSMPSRNVIQISNDLENLRDLLHVLAFSKSCHLPWASG LETLDSLGGVLEASGYSTEVVALSRLQGSLQDMLWQLDLSPGC (SEQ ID NO : 96) | |

## Claims

1. Use of one or more peptides for the manufacture of a preparation for locally modulating satiety of a mammalian subject within the oral cavity of a mammalian subject, wherein said one or more peptides are selected from the group consisting of gastrointestinal and pancreatic peptide hormones, and salivary peptides.

2. The use according to claim 1, wherein said one or more peptides exhibit a satiety correlation coefficient |r| in the range of 0.70 - 0.95 from SELDI-TOF-MS analysis.

3. The use according to claim 1 or 2, for inducing satiety.

4. The use according to claim 1 or 2, for reducing satiety.

5. The use according to claim 1 or 2, wherein said gastrointestinal- and/or pancreatic peptide hormones are selected from the group consisting of insulin α chain, insulin β chain, insulin-like peptides 1-3, somatostatin, gastrin, cholecystokinin (CCK), secretin, motilin, vasoactive intestinal peptide (VIP), gastric inhibitory polypeptide, enteroglucagon, peptide YY (PYY), pancreatic hormone, pancreatic icosapeptide, ghrelin, glucagon, glucagon-like peptide 1 (GLP-1), glucagon-like peptide 2 (GLP-2), glicentin, glucose-dependent insulinotropic polypeptide (GIP) and amylin.

6. The use according to claim 1 or 2, wherein said salivary peptides are selected from the group consisting of defensins, somatomedins, histatins and leptin.

7. The use according to any one of the preceding claims, wherein said composition is incorporated in a spray, aerosol, toothpaste, or chewing gum.

8. A composition for administration to the oral cavity of a mammalian subject, said composition comprising a satiety-modulating amount of one or more salivary peptides, provided that said composition is not saliva.

9. The composition according to claim 9, wherein said salivary peptides are selected from the group consisting of defensins, somatomedins, histatins and leptin.

10. A composition for administration to the oral cavity of a mammalian subject, said composition comprising a satiety-modulating amount of one or more gastrointestinal- and/or pancreatic hormone peptides, whereby said peptides are selected from the group consisting of insulin α chain, insulin β chain, insulin-like peptides 1-3, somatostatin, gastrin, secretin, motilin, vasoactive intestinal peptide (VIP), gastric inhibitory polypeptide, enteroglucagon, pancreatic hormone, pancreatic icosapeptide, ghrelin, glucagon, glucagon-like peptide 2 (GLP-2), glicentin, glucose-dependent insulinotropic polypeptide (GIP), provided that said composition is not saliva.

11. The composition according to any one of claims 8 - 10, wherein said composition is a liquid or semi-liquid composition and/or wherein said peptides are freely dispersed within said composition.

12. The composition according to any one of claims 8 - 11, being a food or feed supplement or composition.

13. The composition according to claim 12, being incorporated in a spray, aerosol, toothpaste, or chewing gum.


SEQUENCE LISTING

<110>  Stichting Top Institute Food and Nutrition

<120>  Satiety manipulating compositions

<130>  P601693EP

<160>  96

<170>  PatentIn version 3.3

<210>  1
<211>  113
<212>  PRT
<213>  unknown

<220>
<223>  O15130 - neuropeptide SF

<400>  1

Met Asp Ser Arg Gln Ala Ala Ala Leu Leu Val Leu Leu Leu Leu Ile
1               5                   10                  15

Asp Gly Gly Cys Ala Glu Gly Pro Gly Gly Gln Gln Glu Asp Gln Leu
            20                  25                  30

Ser Ala Glu Glu Asp Ser Glu Pro Leu Pro Pro Gln Asp Ala Gln Thr
        35                  40                  45

Ser Gly Ser Leu Leu His Tyr Leu Leu Gln Ala Met Glu Arg Pro Gly
    50                  55                  60

Arg Ser Gln Ala Phe Leu Phe Gln Pro Gln Arg Phe Gly Arg Asn Thr
65                  70                  75                  80

Gln Gly Ser Trp Arg Asn Glu Trp Leu Ser Pro Arg Ala Gly Glu Gly
                85                  90                  95

Leu Asn Ser Gln Phe Trp Ser Leu Ala Ala Pro Gln Arg Phe Gly Lys
            100                 105                 110

Lys

<210>  2
<211>  145
<212>  PRT
<213>  unknown

<220>
<223>  Q15726 - kisspeptin 14

<400>  2

<210>  3
<211>  131
<212>  PRT
<213>  unknown

<220>
<223>  P51460 - insulin like 3A chain

<400>  3

Met Asp Pro Arg Leu Pro Ala Trp Ala Leu Val Leu Leu Gly Pro Ala
1               5                   10                  15

Leu Val Phe Ala Leu Gly Pro Ala Pro Thr Pro Glu Met Arg Glu Lys
            20                  25                  30

Leu Cys Gly His His Phe Val Arg Ala Leu Val Arg Val Cys Gly Gly
        35                  40                  45

Pro Arg Trp Ser Thr Glu Ala Arg Arg Pro Ala Ala Gly Gly Asp Arg
    50                  55                  60

Glu Leu Leu Gln Trp Leu Glu Arg Arg His Leu Leu His Gly Leu Val
65                  70                  75                  80

Ala Asp Ser Asn Leu Thr Leu Gly Pro Gly Leu Gln Pro Leu Pro Gln
                85                  90                  95

Thr Ser His His His Arg His His Arg Ala Ala Ala Thr Asn Pro Ala
            100                 105                 110

Arg Tyr Cys Cys Leu Ser Gly Cys Thr Gln Gln Asp Leu Leu Thr Leu
        115                 120                 125

Cys Pro Tyr
    130

<210>  4
<211>  139
<212>  PRT
<213>  unknown

<220>
<223>  Q14641 - earlt placenta insulin-like peptide A

<400>  4

Met Ala Ser Leu Phe Arg Ser Tyr Leu Pro Ala Ile Trp Leu Leu Leu
1               5                   10                  15

Ser Gln Leu Leu Arg Glu Ser Leu Ala Ala Glu Leu Arg Gly Cys Gly
            20                  25                  30

Pro Arg Phe Gly Lys His Leu Leu Ser Tyr Cys Pro Met Pro Glu Lys
        35                  40                  45

Thr Phe Thr Thr Thr Pro Gly Gly Trp Leu Leu Glu Ser Gly Arg Pro
    50                  55                  60

Lys Glu Met Val Ser Thr Ser Asn Asn Lys Asp Gly Gln Ala Leu Gly
65                  70                  75                  80

Thr Thr Ser Glu Phe Ile Pro Asn Leu Ser Pro Glu Leu Lys Lys Pro
                85                  90                  95

Leu Ser Glu Gly Gln Pro Ser Leu Lys Lys Ile Ile Leu Ser Arg Lys
            100                 105                 110

Lys Arg Ser Gly Arg His Arg Phe Asp Pro Phe Cys Cys Glu Val Ile
        115                 120                 125

Cys Asp Asp Gly Thr Ser Val Lys Leu Cys Thr
    130                 135

<210>  5
<211>  379
<212>  PRT
<213>  unknown

<220>
<223>  Q9H300 - P-beta

<400>  5

Met Ala Trp Arg Gly Trp Ala Gln Arg Gly Trp Gly Cys Gly Gln Ala

1               5                   10                  15

Trp Gly Ala Ser Val Gly Gly Arg Ser Cys Glu Glu Leu Thr Ala Val
            20                  25                  30

Leu Thr Pro Pro Gln Leu Leu Gly Arg Arg Phe Asn Phe Phe Ile Gln
        35                  40                  45

Gln Lys Cys Gly Phe Arg Lys Ala Pro Arg Lys Val Glu Pro Arg Arg
    50                  55                  60

Ser Asp Pro Gly Thr Ser Gly Glu Ala Tyr Lys Arg Ser Ala Leu Ile
65                  70                  75                  80

Pro Pro Val Glu Glu Thr Val Phe Tyr Pro Ser Pro Tyr Pro Ile Arg
                85                  90                  95

Ser Leu Ile Lys Pro Leu Phe Phe Thr Val Gly Phe Thr Gly Cys Ala
            100                 105                 110

Phe Gly Ser Ala Ala Ile Trp Gln Tyr Glu Ser Leu Lys Ser Arg Val
        115                 120                 125

Gln Ser Tyr Phe Asp Gly Ile Lys Ala Asp Trp Leu Asp Ser Ile Arg
    130                 135                 140

Pro Gln Lys Glu Gly Asp Phe Arg Lys Glu Ile Asn Lys Trp Trp Asn
145                 150                 155                 160

Asn Leu Ser Asp Gly Gln Arg Thr Val Thr Gly Ile Ile Ala Ala Asn
                165                 170                 175

Val Leu Val Phe Cys Leu Trp Arg Val Pro Ser Leu Gln Arg Thr Met
            180                 185                 190

Ile Arg Tyr Phe Thr Ser Asn Pro Ala Ser Lys Val Leu Cys Ser Pro
        195                 200                 205

Met Leu Leu Ser Thr Phe Ser His Phe Ser Leu Phe His Met Ala Ala
    210                 215                 220

Asn Met Tyr Val Leu Trp Ser Phe Ser Ser Ser Ile Val Asn Ile Leu
225                 230                 235                 240

Gly Gln Glu Gln Phe Met Ala Val Tyr Leu Ser Ala Gly Val Ile Ser
                245                 250                 255

Asn Phe Val Ser Tyr Val Gly Lys Val Ala Thr Gly Arg Tyr Gly Pro
            260                 265                 270

Ser Leu Gly Ala Ser Gly Ala Ile Met Thr Val Leu Ala Ala Val Cys
        275                 280                 285

Thr Lys Ile Pro Glu Gly Arg Leu Ala Ile Ile Phe Leu Pro Met Phe
    290                 295                 300

Thr Phe Thr Ala Gly Asn Ala Leu Lys Ala Ile Ile Ala Met Asp Thr
305                 310                 315                 320

Ala Gly Met Ile Leu Gly Trp Lys Phe Phe Asp His Ala Ala His Leu
                325                 330                 335

Gly Gly Ala Leu Phe Gly Ile Trp Tyr Val Thr Tyr Gly His Glu Leu
            340                 345                 350

Ile Trp Lys Asn Arg Glu Pro Leu Val Lys Ile Trp His Glu Ile Arg
        355                 360                 365

Thr Asn Gly Pro Lys Lys Gly Gly Gly Ser Lys
    370                 375

<210>  6
<211>  117
<212>  PRT
<213>  unknown

<220>
<223>  Q9UBU3 - ghrelin 28

<400>  6

Met Pro Ser Pro Gly Thr Val Cys Ser Leu Leu Leu Leu Gly Met Leu
1               5                   10                  15

Trp Leu Asp Leu Ala Met Ala Gly Ser Ser Phe Leu Ser Pro Glu His
            20                  25                  30

Gln Arg Val Gln Gln Arg Lys Glu Ser Lys Lys Pro Pro Ala Lys Leu
        35                  40                  45

Gln Pro Arg Ala Leu Ala Gly Trp Leu Arg Pro Glu Asp Gly Gly Gln
    50                  55                  60

Ala Glu Gly Ala Glu Asp Glu Leu Glu Val Arg Phe Asn Ala Pro Phe
65                  70                  75                  80

Asp Val Gly Ile Lys Leu Ser Gly Val Gln Tyr Gln Gln His Ser Gln
                85                  90                  95

Ala Leu Gly Lys Phe Leu Gln Asp Ile Leu Trp Glu Glu Ala Lys Glu
            100                 105                 110

Ala Pro Ala Asp Lys
        115

<210>  7

<211>  94
<212>  PRT
<213>  unknown

<220>
<223>  P59665 - neutrophil defensin

<400>  7

Met Arg Thr Leu Ala Ile Leu Ala Ala Ile Leu Leu Val Ala Leu Gln
1               5                   10                  15

Ala Gln Ala Glu Pro Leu Gln Ala Arg Ala Asp Glu Val Ala Ala Ala
            20                  25                  30

Pro Glu Gln Ile Ala Ala Asp Ile Pro Glu Val Val Val Ser Leu Ala
        35                  40                  45

Trp Asp Glu Ser Leu Ala Pro Lys His Pro Gly Ser Arg Lys Asn Met
    50                  55                  60

Ala Cys Tyr Cys Arg Ile Pro Ala Cys Ile Ala Gly Glu Arg Arg Tyr
65                  70                  75                  80

Gly Thr Cys Ile Tyr Gln Gly Arg Leu Trp Ala Phe Cys Cys
                85                  90


<210>  8
<211>  33
<212>  PRT
<213>  unknown

<220>
<223>  P59042 - hypothetical protein

<400>  8

Met Gly Pro Pro Gly Asp Glu Glu Pro Leu Glu Gly Pro Glu Leu His
1               5                   10                  15

Val Leu Met Ile Asn Ala Pro Ser Val Leu Ala Gly Phe Ser Asn Ala
            20                  25                  30

Ser



<210>  9
<211>  110
<212>  PRT
<213>  unknown

<220>
<223>  P01308 - insulin B chain

<400>  9

<210>  10
<211>  57
<212>  PRT
<213>  unknown

<220>
<223>  P15515 - histatin-1

<400>  10

Met Lys Phe Phe Val Phe Ala Leu Val Leu Ala Leu Met Ile Ser Met
1               5                   10                  15

Ile Ser Ala Asp Ser His Glu Lys Arg His His Gly Tyr Arg Arg Lys
            20                  25                  30

Phe His Glu Lys His His Ser His Arg Glu Phe Pro Phe Tyr Gly Asp
        35                  40                  45

Tyr Gly Ser Asn Tyr Leu Tyr Asp Asn
    50                  55

<210>  11
<211>  180
<212>  PRT
<213>  unknown

<220>
<223>  P01275 - glucagon

<400>  11

Met Lys Ser Ile Tyr Phe Val Ala Gly Leu Phe Val Met Leu Val Gln
1               5                   10                  15

Gly Ser Trp Gln Arg Ser Leu Gln Asp Thr Glu Glu Lys Ser Arg Ser
            20                  25                  30

Phe Ser Ala Ser Gln Ala Asp Pro Leu Ser Asp Pro Asp Gln Met Asn

35                  40                  45

Glu Asp Lys Arg His Ser Gln Gly Thr Phe Thr Ser Asp Tyr Ser Lys
    50                  55                  60

Tyr Leu Asp Ser Arg Arg Ala Gln Asp Phe Val Gln Trp Leu Met Asn
65                  70                  75                  80

Thr Lys Arg Asn Arg Asn Asn Ile Ala Lys Arg His Asp Glu Phe Glu
                85                  90                  95

Arg His Ala Glu Gly Thr Phe Thr Ser Asp Val Ser Ser Tyr Leu Glu
            100                 105                 110

Gly Gln Ala Ala Lys Glu Phe Ile Ala Trp Leu Val Lys Gly Arg Gly
        115                 120                 125

Arg Arg Asp Phe Pro Glu Glu Val Ala Ile Val Glu Glu Leu Gly Arg
    130                 135                 140

Arg His Ala Asp Gly Ser Phe Ser Asp Glu Met Asn Thr Ile Leu Asp
145                 150                 155                 160

Asn Leu Ala Ala Arg Asp Phe Ile Asn Trp Leu Ile Gln Thr Lys Ile
                165                 170                 175

Thr Asp Arg Lys
            180

<210>  12
<211>  97
<212>  PRT
<213>  unknown

<220>
<223>  P01303 - C- flanking peptide of NPY

<400>  12

Met Leu Gly Asn Lys Arg Leu Gly Leu Ser Gly Leu Thr Leu Ala Leu
1               5                   10                  15

Ser Leu Leu Val Cys Leu Gly Ala Leu Ala Glu Ala Tyr Pro Ser Lys
            20                  25                  30

Pro Asp Asn Pro Gly Glu Asp Ala Pro Ala Glu Asp Met Ala Arg Tyr
        35                  40                  45

Tyr Ser Ala Leu Arg His Tyr Ile Asn Leu Ile Thr Arg Gln Arg Tyr
    50                  55                  60

Gly Lys Arg Ser Ser Pro Glu Thr Leu Ile Ser Asp Leu Leu Met Arg
65                  70                  75                  80

Glu Ser Thr Glu Asn Val Pro Arg Thr Arg Leu Glu Asp Pro Ala Met
85                  90                  95

Trp

<210>  13
<211>  97
<212>  PRT
<213>  unknown

<220>
<223>  P10082 - peptide YY

<400>  13

Met Val Phe Val Arg Arg Pro Trp Pro Ala Leu Thr Thr Val Leu Leu
1               5                   10                  15

Ala Leu Leu Val Cys Leu Gly Ala Leu Val Asp Ala Tyr Pro Ile Lys
            20                  25                  30

Pro Glu Ala Pro Gly Glu Asp Ala Ser Pro Glu Glu Leu Asn Arg Tyr
        35                  40                  45

Tyr Ala Ser Leu Arg His Tyr Leu Asn Leu Val Thr Arg Gln Arg Tyr
    50                  55                  60

Gly Lys Arg Asp Gly Pro Asp Thr Leu Leu Ser Lys Thr Phe Phe Pro
65                  70                  75                  80

Asp Gly Glu Asp Arg Pro Val Arg Ser Arg Ser Glu Gly Pro Asp Leu
                85                  90                  95

Trp

<210>  14
<211>  41
<212>  PRT
<213>  unknown

<220>
<223>  Q07815 - Bax protein

<400>  14

Met Asp Gly Ser Gly Glu Gln Pro Arg Gly Gly Val Ser Ser Arg Ile
1               5                   10                  15

Glu Gln Gly Glu Trp Gly Gly Arg His Pro Ser Trp Pro Trp Thr Arg
            20                  25                  30

Cys Leu Arg Met Arg Pro Pro Arg Ser
        35                  40

<210>  15

<211>  124
<212>  PRT
<213>  unknown

<220>
<223>  P55089 - urocortin

<400>  15

Met Arg Gln Ala Gly Arg Ala Ala Leu Leu Ala Ala Leu Leu Leu Leu
1               5                   10                  15

Val Gln Leu Cys Pro Gly Ser Ser Gln Arg Ser Pro Glu Ala Ala Gly
            20                  25                  30

Val Gln Asp Pro Ser Leu Arg Trp Ser Pro Gly Ala Arg Asn Gln Gly
        35                  40                  45

Gly Gly Ala Arg Ala Leu Leu Leu Leu Leu Ala Glu Arg Phe Pro Arg
    50                  55                  60

Arg Ala Gly Pro Gly Arg Leu Gly Leu Gly Thr Ala Gly Glu Arg Pro
65                  70                  75                  80

Arg Arg Asp Asn Pro Ser Leu Ser Ile Asp Leu Thr Phe His Leu Leu
                85                  90                  95

Arg Thr Leu Leu Glu Leu Ala Arg Thr Gln Ser Gln Arg Glu Arg Ala
            100                 105                 110

Glu Gln Asn Arg Ile Ile Phe Asp Ser Val Gly Lys
        115                 120


<210>  16
<211>  69
<212>  PRT
<213>  unknown

<220>
<223>  P10176 cytochrome c oxidase

<400>  16

Met Ser Val Leu Thr Pro Leu Leu Leu Arg Gly Leu Thr Gly Ser Ala
1               5                   10                  15

Arg Arg Leu Pro Val Pro Arg Ala Lys Ile His Ser Leu Pro Pro Glu
            20                  25                  30

Gly Lys Leu Gly Ile Met Glu Leu Ala Val Gly Leu Thr Ser Cys Phe
        35                  40                  45

Val Thr Phe Leu Leu Pro Ala Gly Trp Ile Leu Ser His Leu Glu Thr
    50                  55                  60

Tyr Arg Arg Pro Glu
65

<210>  17
<211>  44
<212>  PRT
<213>  unknown

<220>
<223>  P63313 - thymosin beta-10

<400>  17

Met Ala Asp Lys Pro Asp Met Gly Glu Ile Ala Ser Phe Asp Lys Ala
1               5                   10                  15

Lys Leu Lys Lys Thr Glu Thr Gln Glu Lys Asn Thr Leu Pro Thr Lys
            20                  25                  30

Glu Thr Ile Glu Gln Glu Lys Arg Ser Glu Ile Ser
        35                  40

<210>  18
<211>  44
<212>  PRT
<213>  unknown

<220>
<223>  P62328 - thymosin beta-4

<400>  18

Met Ser Asp Lys Pro Asp Met Ala Glu Ile Glu Lys Phe Asp Lys Ser
1               5                   10                  15

Lys Leu Lys Lys Thr Glu Thr Gln Glu Lys Asn Pro Leu Pro Ser Lys
            20                  25                  30

Glu Thr Ile Glu Gln Glu Lys Gln Ala Gly Glu Ser
        35                  40

<210>  19
<211>  111
<212>  PRT
<213>  unknown

<220>
<223>  Q9BYW3 - beta defensin 126

<400>  19

Met Lys Ser Leu Leu Phe Thr Leu Ala Val Phe Met Leu Leu Ala Gln
1               5                   10                  15

Leu Val Ser Gly Asn Trp Tyr Val Lys Lys Cys Leu Asn Asp Val Gly
            20                  25                  30

Ile Cys Lys Lys Lys Cys Lys Pro Glu Glu Met His Val Lys Asn Gly
        35                  40                  45

Trp Ala Met Cys Gly Lys Gln Arg Asp Cys Cys Val Pro Ala Asp Arg

50                  55                  60

Arg Ala Asn Tyr Pro Val Phe Cys Val Gln Thr Lys Thr Thr Arg Ile
65                  70                  75                  80

Ser Thr Val Thr Ala Thr Thr Ala Thr Thr Thr Leu Met Met Thr Thr
                85                  90                  95

Ala Ser Met Ser Ser Met Ala Pro Thr Pro Val Ser Pro Thr Gly
            100                 105                 110

<210>  20
<211>  478
<212>  PRT
<213>  unknown

<220>
<223>  P04004 - somatomedin B

<400>  20

Met Ala Pro Leu Arg Pro Leu Leu Ile Leu Ala Leu Leu Ala Trp Val
1               5                   10                  15

Ala Leu Ala Asp Gln Glu Ser Cys Lys Gly Arg Cys Thr Glu Gly Phe
            20                  25                  30

Asn Val Asp Lys Lys Cys Gln Cys Asp Glu Leu Cys Ser Tyr Tyr Gln
        35                  40                  45

Ser Cys Cys Thr Asp Tyr Thr Ala Glu Cys Lys Pro Gln Val Thr Arg
    50                  55                  60

Gly Asp Val Phe Thr Met Pro Glu Asp Glu Tyr Thr Val Tyr Asp Asp
65                  70                  75                  80

Gly Glu Glu Lys Asn Asn Ala Thr Val His Glu Gln Val Gly Gly Pro
                85                  90                  95

Ser Leu Thr Ser Asp Leu Gln Ala Gln Ser Lys Gly Asn Pro Glu Gln
            100                 105                 110

Thr Pro Val Leu Lys Pro Glu Glu Glu Ala Pro Ala Pro Glu Val Gly
        115                 120                 125

Ala Ser Lys Pro Glu Gly Ile Asp Ser Arg Pro Glu Thr Leu His Pro
    130                 135                 140

Gly Arg Pro Gln Pro Pro Ala Glu Glu Glu Leu Cys Ser Gly Lys Pro
145                 150                 155                 160

Phe Asp Ala Phe Thr Asp Leu Lys Asn Gly Ser Leu Phe Ala Phe Arg
                165                 170                 175

Gly Gln Tyr Cys Tyr Glu Leu Asp Glu Lys Ala Val Arg Pro Gly Tyr
            180                 185                 190

Pro Lys Leu Ile Arg Asp Val Trp Gly Ile Glu Gly Pro Ile Asp Ala
        195                 200                 205

Ala Phe Thr Arg Ile Asn Cys Gln Gly Lys Thr Tyr Leu Phe Lys Gly
    210                 215                 220

Ser Gln Tyr Trp Arg Phe Glu Asp Gly Val Leu Asp Pro Asp Tyr Pro
225                 230                 235                 240

Arg Asn Ile Ser Asp Gly Phe Asp Gly Ile Pro Asp Asn Val Asp Ala
                245                 250                 255

Ala Leu Ala Leu Pro Ala His Ser Tyr Ser Gly Arg Glu Arg Val Tyr
            260                 265                 270

Phe Phe Lys Gly Lys Gln Tyr Trp Glu Tyr Gln Phe Gln His Gln Pro
        275                 280                 285

Ser Gln Glu Glu Cys Glu Gly Ser Ser Leu Ser Ala Val Phe Glu His
    290                 295                 300

Phe Ala Met Met Gln Arg Asp Ser Trp Glu Asp Ile Phe Glu Leu Leu
305                 310                 315                 320

Phe Trp Gly Arg Thr Ser Ala Gly Thr Arg Gln Pro Gln Phe Ile Ser
                325                 330                 335

Arg Asp Trp His Gly Val Pro Gly Gln Val Asp Ala Ala Met Ala Gly
            340                 345                 350

Arg Ile Tyr Ile Ser Gly Met Ala Pro Arg Pro Ser Leu Ala Lys Lys
        355                 360                 365

Gln Arg Phe Arg His Arg Asn Arg Lys Gly Tyr Arg Ser Gln Arg Gly
    370                 375                 380

His Ser Arg Gly Arg Asn Gln Asn Ser Arg Arg Pro Ser Arg Ala Thr
385                 390                 395                 400

Trp Leu Ser Leu Phe Ser Ser Glu Glu Ser Asn Leu Gly Ala Asn Asn
                405                 410                 415

Tyr Asp Asp Tyr Arg Met Asp Trp Leu Val Pro Ala Thr Cys Glu Pro
            420                 425                 430

Ile Gln Ser Val Phe Phe Phe Ser Gly Asp Lys Tyr Tyr Arg Val Asn
        435                 440                 445

Leu Arg Thr Arg Arg Val Asp Thr Val Asp Pro Pro Tyr Pro Arg Ser

450                 455                 460

Ile Ala Gln Tyr Trp Leu Gly Cys Pro Ala Pro Gly His Leu
465                 470                 475

<210>  21
<211>  593
<212>  PRT
<213>  unknown

<220>
<223>  P28799 - granulin-5

<400>  21

<210> 22
<211> 93
<212> PRT
<213> unknown

<220>
<223> 000626 - small inducible cytokine A22

<400> 22

Met Ala Arg Leu Gln Thr Ala Leu Leu Val Val Leu Val Leu Leu Ala
1               5                   10                  15

Val Ala Leu Gln Ala Thr Glu Ala Gly Pro Tyr Gly Ala Asn Met Glu
            20                  25                  30

Asp Ser Val Cys Cys Arg Asp Tyr Val Arg Tyr Arg Leu Pro Leu Arg
        35                  40                  45

Val Val Lys His Phe Tyr Trp Thr Ser Asp Ser Cys Pro Arg Pro Gly
    50                  55                  60

Val Val Leu Leu Thr Phe Arg Asp Lys Glu Ile Cys Ala Asp Pro Arg
65                  70                  75                  80

Val Pro Trp Val Lys Met Ile Leu Asn Lys Leu Ser Gln
                85                  90

<210> 23
<211> 180
<212> PRT
<213> unknown

<220>
<223>  P01275 - glicentin

<400>  23

Met Lys Ser Ile Tyr Phe Val Ala Gly Leu Phe Val Met Leu Val Gln
1               5                   10                  15

Gly Ser Trp Gln Arg Ser Leu Gln Asp Thr Glu Glu Lys Ser Arg Ser
            20                  25                  30

Phe Ser Ala Ser Gln Ala Asp Pro Leu Ser Asp Pro Asp Gln Met Asn
        35                  40                  45

Glu Asp Lys Arg His Ser Gln Gly Thr Phe Thr Ser Asp Tyr Ser Lys
    50                  55                  60

Tyr Leu Asp Ser Arg Arg Ala Gln Asp Phe Val Gln Trp Leu Met Asn
65                  70                  75                  80

Thr Lys Arg Asn Arg Asn Asn Ile Ala Lys Arg His Asp Glu Phe Glu
                85                  90                  95

Arg His Ala Glu Gly Thr Phe Thr Ser Asp Val Ser Ser Tyr Leu Glu
            100                 105                 110

Gly Gln Ala Ala Lys Glu Phe Ile Ala Trp Leu Val Lys Gly Arg Gly
        115                 120                 125

Arg Arg Asp Phe Pro Glu Glu Val Ala Ile Val Glu Glu Leu Gly Arg
    130                 135                 140

Arg His Ala Asp Gly Ser Phe Ser Asp Glu Met Asn Thr Ile Leu Asp
145                 150                 155                 160

Asn Leu Ala Ala Arg Asp Phe Ile Asn Trp Leu Ile Gln Thr Lys Ile
                165                 170                 175

Thr Asp Arg Lys
            180


<210>  24
<211>  99
<212>  PRT
<213>  unknown

<220>
<223>  P10145 - IL8

<400>  24

Met Thr Ser Lys Leu Ala Val Ala Leu Leu Ala Ala Phe Leu Ile Ser
1               5                   10                  15

Ala Ala Leu Cys Glu Gly Ala Val Leu Pro Arg Ser Ala Lys Glu Leu

20                  25                  30

Arg Cys Gln Cys Ile Lys Thr Tyr Ser Lys Pro Phe His Pro Lys Phe
        35                  40                  45

Ile Lys Glu Leu Arg Val Ile Glu Ser Gly Pro His Cys Ala Asn Thr
    50                  55                  60

Glu Ile Ile Val Lys Leu Ser Asp Gly Arg Glu Leu Cys Leu Asp Pro
65                  70                  75                  80

Lys Glu Asn Trp Val Gln Arg Val Val Glu Lys Phe Leu Lys Arg Ala
                85                  90                  95

Glu Asn Ser

<210>  25
<211>  128
<212>  PRT
<213>  unknown

<220>
<223>  P02775 - platelet basic protein

<400>  25

Met Ser Leu Arg Leu Asp Thr Thr Pro Ser Cys Asn Ser Ala Arg Pro
1               5                   10                  15

Leu His Ala Leu Gln Val Leu Leu Leu Leu Ser Leu Leu Leu Thr Ala
            20                  25                  30

Leu Ala Ser Ser Thr Lys Gly Gln Thr Lys Arg Asn Leu Ala Lys Gly
        35                  40                  45

Lys Glu Glu Ser Leu Asp Ser Asp Leu Tyr Ala Glu Leu Arg Cys Met
    50                  55                  60

Cys Ile Lys Thr Thr Ser Gly Ile His Pro Lys Asn Ile Gln Ser Leu
65                  70                  75                  80

Glu Val Ile Gly Lys Gly Thr His Cys Asn Gln Val Glu Val Ile Ala
                85                  90                  95

Thr Leu Lys Asp Gly Arg Lys Ile Cys Leu Asp Pro Asp Ala Pro Arg
            100                 105                 110

Ile Lys Lys Ile Val Gln Lys Lys Leu Ala Gly Asp Glu Ser Ala Asp
        115                 120                 125

<210>  26
<211>  102
<212>  PRT
<213>  unknown

<220>
<223>  P04554 - basic nuclear protein HPS2

<400>  26

Met Val Arg Tyr Arg Val Arg Ser Leu Ser Glu Arg Ser His Glu Val
1               5                   10                  15

Tyr Arg Gln Gln Leu His Gly Gln Glu Gln Gly His His Gly Gln Glu
            20                  25                  30

Glu Gln Gly Leu Ser Pro Glu His Val Glu Val Tyr Glu Arg Thr His
        35                  40                  45

Gly Gln Ser His Tyr Arg Arg Arg His Cys Ser Arg Arg Arg Leu His
    50                  55                  60

Arg Ile His Arg Arg Gln His Arg Ser Cys Arg Arg Arg Lys Arg Arg
65                  70                  75                  80

Ser Cys Arg His Arg Arg Arg His Arg Arg Gly Cys Arg Thr Arg Lys
                85                  90                  95

Arg Thr Cys Arg Arg His
            100

<210>  27
<211>  78
<212>  PRT
<213>  unknown

<220>
<223>  Q9H4G8 zinc finger containing protein

<400>  27

Met Ala Val Phe His Asp Glu Val Glu Ile Glu Asp Phe Gln Tyr Asp
1               5                   10                  15

Glu Asp Ser Glu Thr Tyr Phe Cys Pro Cys Pro Cys Gly Asp Asn Phe
            20                  25                  30

Ser Ile Thr Lys Glu Glu Leu Glu Asn Gly Glu Gly Val Ala Met Cys
        35                  40                  45

Pro Gly Cys Ser Leu Ile Ile Lys Val Ile Tyr Asp Lys Asp Gln Phe
    50                  55                  60

Ala Cys Gly Glu Thr Val Pro Val Pro Ser Val Asn Lys Glu
65                  70                  75

<210>  28
<211>  101
<212>  PRT
<213>  unknown

<220>
<223>  P02655 - apolipoprotein C-II

<400>  28

Met Gly Thr Arg Leu Leu Pro Ala Leu Phe Leu Val Leu Leu Val Leu
1               5                   10                  15

Gly Phe Glu Val Gln Gly Thr Gln Gln Pro Gln Gln Asp Glu Met Pro
            20                  25                  30

Ser Pro Thr Phe Leu Thr Gln Val Lys Glu Ser Leu Ser Ser Tyr Trp
        35                  40                  45

Glu Ser Ala Lys Thr Ala Ala Gln Asn Leu Tyr Glu Lys Thr Tyr Leu
    50                  55                  60

Pro Ala Val Asp Glu Lys Leu Arg Asp Leu Tyr Ser Lys Ser Thr Ala
65                  70                  75                  80

Ala Met Ser Thr Tyr Thr Gly Ile Phe Thr Asp Gln Val Leu Ser Val
                85                  90                  95

Leu Lys Gly Glu Glu
            100


<210>  29
<211>  99
<212>  PRT
<213>  unknown

<220>
<223>  P80075 - small inducible cytokine A8

<400>  29

Met Lys Val Ser Ala Ala Leu Leu Cys Leu Leu Leu Met Ala Ala Thr
1               5                   10                  15

Phe Ser Pro Gln Gly Leu Ala Gln Pro Asp Ser Val Ser Ile Pro Ile
            20                  25                  30

Thr Cys Cys Phe Asn Val Ile Asn Arg Lys Ile Pro Ile Gln Arg Leu
        35                  40                  45

Glu Ser Tyr Thr Arg Ile Thr Asn Ile Gln Cys Pro Lys Glu Ala Val
    50                  55                  60

Ile Phe Lys Thr Lys Arg Gly Lys Glu Val Cys Ala Asp Pro Lys Glu
65                  70                  75                  80

Arg Trp Val Arg Asp Ser Met Lys His Leu Asp Gln Ile Phe Gln Asn
                85                  90                  95

Leu Lys Pro

<210>  30
<211>  99
<212>  PRT
<213>  unknown

<220>
<223>  P10145 - IL8

<400>  30

Met Thr Ser Lys Leu Ala Val Ala Leu Leu Ala Ala Phe Leu Ile Ser
1               5                   10                  15

Ala Ala Leu Cys Glu Gly Ala Val Leu Pro Arg Ser Ala Lys Glu Leu
            20                  25                  30

Arg Cys Gln Cys Ile Lys Thr Tyr Ser Lys Pro Phe His Pro Lys Phe
        35                  40                  45

Ile Lys Glu Leu Arg Val Ile Glu Ser Gly Pro His Cys Ala Asn Thr
    50                  55                  60

Glu Ile Ile Val Lys Leu Ser Asp Gly Arg Glu Leu Cys Leu Asp Pro
65                  70                  75                  80

Lys Glu Asn Trp Val Gln Arg Val Val Glu Lys Phe Leu Lys Arg Ala
                85                  90                  95

Glu Asn Ser



<210>  31
<211>  810
<212>  PRT
<213>  unknown

<220>
<223>  P00747 - activation peptide

<400>  31

<210>  32
<211>  103
<212>  PRT
<213>  unknown

<220>
<223>  P55000 - secreted Ly6/uPAR related protein

<400>  32

Met Ala Ser Arg Trp Ala Val Gln Leu Leu Leu Val Ala Ala Trp Ser
1               5                   10                  15

Met Gly Cys Gly Glu Ala Leu Lys Cys Tyr Thr Cys Lys Glu Pro Met
            20                  25                  30

Thr Ser Ala Ser Cys Arg Thr Ile Thr Arg Cys Lys Pro Glu Asp Thr
        35                  40                  45

Ala Cys Met Thr Thr Leu Val Thr Val Glu Ala Glu Tyr Pro Phe Asn
    50                  55                  60

Gln Ser Pro Val Val Thr Arg Ser Cys Ser Ser Ser Cys Val Ala Thr
65                  70                  75                  80

Asp Pro Asp Ser Ile Gly Ala Ala His Leu Ile Phe Cys Cys Phe Arg
                85                  90                  95

Asp Leu Cys Asn Ser Glu Leu
            100

<210>  33
<211>  85
<212>  PRT
<213>  unknown

<220>
<223>  P61960 - ubiquitin fold modifier 1

<400>  33

Met Ser Lys Val Ser Phe Lys Ile Thr Leu Thr Ser Asp Pro Arg Leu
1               5                   10                  15

Pro Tyr Lys Val Leu Ser Val Pro Glu Ser Thr Pro Phe Thr Ala Val
            20                  25                  30

Leu Lys Phe Ala Ala Glu Glu Phe Lys Val Pro Ala Ala Thr Ser Ala
        35                  40                  45

Ile Ile Thr Asn Asp Gly Ile Gly Ile Asn Pro Ala Gln Thr Ala Gly
    50                  55                  60

Asn Val Phe Leu Lys His Gly Ser Glu Leu Arg Ile Ile Pro Arg Asp
65                  70                  75                  80

Arg Val Gly Ser Cys
                85

<210>  34
<211>  84
<212>  PRT
<213>  unknown

<220>
<223>  Q3LI70 - keratin associated protein 19-6

<400>  34

Met Arg Tyr Tyr Gly Ser Tyr Tyr Arg Gly Leu Gly Tyr Gly Cys Gly
1               5                   10                  15

Gly Phe Gly Gly Leu Gly Tyr Gly Cys Gly Cys Gly Gly Tyr Arg Tyr
            20                  25                  30

Gly Ser Gly Tyr Gly Gly Tyr Arg Tyr Gly Cys Cys Arg Pro Ser Cys

35                  40                  45

Arg Glu Gly Tyr Gly Phe Ser Gly Phe Thr Lys Lys Leu Ala Asp Ser
    50                  55                  60

His Thr Leu Thr Pro Arg Ser Cys Pro Tyr Ala Ser Ala Arg Arg Thr
65                  70                  75                  80

Leu Phe Ser Met

<210>  35
<211>  80
<212>  PRT
<213>  unknown

<220>
<223>  P62312 - U6 snRNA associated Sm-like protein LSm6

<400>  35

Met Ser Leu Arg Lys Gln Thr Pro Ser Asp Phe Leu Lys Gln Ile Ile
1               5                   10                  15

Gly Arg Pro Val Val Val Lys Leu Asn Ser Gly Val Asp Tyr Arg Gly
            20                  25                  30

Val Leu Ala Cys Leu Asp Gly Tyr Met Asn Ile Ala Leu Glu Gln Thr
        35                  40                  45

Glu Glu Tyr Val Asn Gly Gln Leu Lys Asn Lys Tyr Gly Asp Ala Phe
    50                  55                  60

Ile Arg Gly Asn Asn Val Leu Tyr Ile Ser Thr Gln Lys Arg Arg Met
65                  70                  75                  80

<210>  36
<211>  524
<212>  PRT
<213>  unknown

<220>
<223>  P07602 - saposin D

<400>  36

<210>  37
<211>  87
<212>  PRT
<213>  unknown

<220>
<223>  P59022 - down syndrome critical region 10 protein

<400>  37

Met Gln Ile Val Gln Gly Phe Pro Ala Asp Ala Pro Leu Cys Ala Leu
1               5                   10                  15

Met Trp Thr Cys Ser Phe Leu Leu Pro Gly Leu Gln Thr Glu Thr Pro
            20                  25                  30

Tyr Pro Cys Thr Ser Leu Cys Leu Ser Ser Ser Gln Ser Ala His Pro
        35                  40                  45

Pro Leu Pro Val Arg Val Phe Ser Ala Glu Ser Gly Tyr Gly Ile Pro
    50                  55                  60

Phe Cys Ala Glu Pro Cys Ser Arg Val Thr Val Cys His Leu Gln Ala
65                  70                  75                  80

Val Pro Val Cys Met Pro Val
                85

<210>  38
<211>  87
<212>  PRT
<213>  unknown

<220>
<223>  Q8IUC3 - keratin associated protein 7-1

<400>  38

Met Thr Arg Tyr Phe Cys Cys Gly Ser Tyr Phe Pro Gly Tyr Pro Ile
1               5                   10                  15

Tyr Gly Thr Asn Phe His Gly Thr Phe Arg Ala Thr Pro Leu Asn Cys
            20                  25                  30

Val Val Pro Leu Gly Ser Pro Leu Asn Tyr Gly Cys Gly Cys Asn Gly
        35                  40                  45

Tyr Ser Ser Leu Gly Tyr Ser Phe Gly Gly Ser Asn Ile Asn Asn Leu
    50                  55                  60

Gly Gly Cys Tyr Gly Gly Ser Phe Tyr Arg Pro Trp Gly Ser Gly Ser
65                  70                  75                  80

Gly Phe Gly Tyr Ser Thr Tyr
                85

<210>  39
<211>  84
<212>  PRT
<213>  unknown

<220>
<223>  O95167 - NADH dehydrogenase

<400>  39

Met Ala Ala Arg Val Gly Ala Phe Leu Lys Asn Ala Trp Asp Lys Glu
1               5                   10                  15

Pro Val Leu Val Val Ser Phe Val Val Gly Gly Leu Ala Val Ile Leu
            20                  25                  30

Pro Pro Leu Ser Pro Tyr Phe Lys Tyr Ser Val Met Ile Asn Lys Ala
        35                  40                  45

Thr Pro Tyr Asn Tyr Pro Val Pro Val Arg Asp Asp Gly Asn Met Pro
    50                  55                  60

Asp Val Pro Ser His Pro Gln Asp Pro Gln Gly Pro Ser Leu Glu Trp
65                  70                  75                  80

Leu Lys Lys Leu

<210>  40
<211>  106
<212>  PRT
<213>  unknown

<220>
<223>  Q9UII2 - ATPase inhibitor

<400>  40

Met Ala Val Thr Ala Leu Ala Ala Arg Thr Trp Leu Gly Val Trp Gly
1               5                   10                  15

Val Arg Thr Met Gln Ala Arg Gly Phe Gly Ser Asp Gln Ser Glu Asn
            20                  25                  30

Val Asp Arg Gly Ala Gly Ser Ile Arg Glu Ala Gly Gly Ala Phe Gly
        35                  40                  45

Lys Arg Glu Gln Ala Glu Glu Glu Arg Tyr Phe Arg Ala Gln Ser Arg
    50                  55                  60

Glu Gln Leu Ala Ala Leu Lys Lys His His Glu Glu Glu Ile Val His
65                  70                  75                  80

His Lys Lys Glu Ile Glu Arg Leu Gln Lys Glu Ile Glu Arg His Lys
                85                  90                  95

Gln Lys Ile Lys Met Leu Lys His Asp Asp
            100                 105

<210>  41
<211>  86
<212>  PRT
<213>  unknown

<220>
<223>  Q9P2X8 - uncharacterized protein

<400>  41

Met Leu Cys Val Ser Gly Phe Thr Ser Asn Leu Tyr Ser Ser Lys Lys
1               5                   10                  15

Asp Asp Lys Met Lys Glu Ile Ser Arg Thr Ser Asn Trp Gly Ser Ser
            20                  25                  30

Phe Ser Glu Lys Ser Gly Cys Met Gln Thr His Pro Ser Met Asn Leu
        35                  40                  45

Asp Cys Arg Asp Val Thr Tyr Val Met Asn Leu Leu Leu Ile Ala Ser
    50                  55                  60

Ser Ser Ser Ser Ser Met Thr Lys Lys Lys Ile Val Thr Met Asn Thr
65                  70                  75                  80

Ser Leu Gly Gln Asn Lys
                85

<210>  42
<211>  105
<212>  PRT
<213>  unknown

<220>
<223>  Q9BYP8 - keratin associated protein 17-1

<400>  42

Met Gly Cys Cys Pro Gly Asp Cys Phe Thr Cys Cys Thr Gln Glu Gln
1               5                   10                  15

Asn Cys Cys Glu Glu Cys Cys Cys Gln Pro Gly Cys Cys Gly Cys Cys
            20                  25                  30

Gly Ser Cys Cys Gly Cys Gly Gly Ser Gly Cys Gly Gly Ser Gly Cys
        35                  40                  45

Gly Gly Ser Cys Cys Gly Ser Ser Cys Cys Gly Ser Gly Cys Gly Gly
    50                  55                  60

Cys Gly Gly Cys Gly Gly Cys Gly Gly Gly Cys Cys Gly Ser Ser Cys
65                  70                  75                  80

Cys Gly Ser Ser Cys Cys Gly Ser Gly Cys Cys Gly Pro Val Cys Cys
                85                  90                  95

Gln Pro Thr Pro Ile Cys Asp Thr Lys
            100                 105

<210>  43
<211>  92
<212>  PRT
<213>  unknown

<220>
<223>  Q5T5B0 - late cornified envelope protein 3E

<400>  43

Met Ser Cys Gln Gln Asn Gln Lys Gln Cys Gln Pro Pro Pro Lys Cys
1               5                   10                  15

Pro Ser Pro Lys Cys Pro Pro Lys Asn Pro Val Gln Cys Leu Pro Pro

20                  25                  30

Ala Ser Ser Gly Cys Ala Pro Ser Ser Gly Gly Cys Gly Pro Ser Ser
        35                  40                  45

Glu Gly Gly Cys Phe Leu Asn His His Arg Arg His His Arg Cys Arg
    50                  55                  60

Arg Gln Arg Ser Asn Ser Cys Asp Arg Gly Ser Gly Gln Gln Gly Gly
65                  70                  75                  80

Gly Ser Gly Cys Cys His Gly Ser Gly Gly Cys Cys
                85                  90

<210>  44
<211>  88
<212>  PRT
<213>  unknown

<220>
<223>  O75324 - stannin

<400>  44

Met Ser Ile Met Asp His Ser Pro Thr Thr Gly Val Val Thr Val Ile
1               5                   10                  15

Val Ile Leu Ile Ala Ile Ala Ala Leu Gly Ala Leu Ile Leu Gly Cys
            20                  25                  30

Trp Cys Tyr Leu Arg Leu Gln Arg Ile Ser Gln Ser Glu Asp Glu Glu
        35                  40                  45

Ser Ile Val Gly Asp Gly Glu Thr Lys Glu Pro Phe Leu Leu Val Gln
    50                  55                  60

Tyr Ser Ala Lys Gly Pro Cys Val Glu Arg Lys Ala Lys Leu Met Thr
65                  70                  75                  80

Pro Asn Gly Pro Glu Val His Gly
                85

<210>  45
<211>  87
<212>  PRT
<213>  unknown

<220>
<223>  P18545 - retinal rod rhodopsin sensitive cGMP PDE gamma subunit

<400>  45

Met Asn Leu Glu Pro Pro Lys Ala Glu Phe Arg Ser Ala Thr Arg Val
1               5                   10                  15

Ala Gly Gly Pro Val Thr Pro Arg Lys Gly Pro Pro Lys Phe Lys Gln
            20                  25                  30

Arg Gln Thr Arg Gln Phe Lys Ser Lys Pro Pro Lys Lys Gly Val Gln
        35                  40                  45

Gly Phe Gly Asp Asp Ile Pro Gly Met Glu Gly Leu Gly Thr Asp Ile
    50                  55                  60

Thr Val Ile Cys Pro Trp Glu Ala Phe Asn His Leu Glu Leu His Glu
65                  70                  75                  80

Leu Ala Gln Tyr Gly Ile Ile
                85

<210>  46
<211>  85
<212>  PRT
<213>  unknown

<220>
<223>  Q7Z4G1 - COMM domain containing protein

<400>  46

Met Glu Ala Ser Ser Glu Pro Pro Leu Asp Ala Lys Ser Asp Val Thr
1               5                   10                  15

Asn Gln Leu Val Asp Phe Gln Trp Lys Leu Gly Met Ala Val Ser Ser
            20                  25                  30

Asp Thr Cys Arg Ser Leu Lys Tyr Pro Tyr Val Ala Val Met Leu Lys
        35                  40                  45

Val Ala Asp His Ser Gly Gln Val Lys Thr Lys Cys Phe Glu Met Thr
    50                  55                  60

Ile Pro Gln Phe Gln Asn Phe Tyr Arg Gln Phe Lys Glu Ile Ala Ala
65                  70                  75                  80

Val Ile Glu Thr Val
                85

<210>  47
<211>  156
<212>  PRT
<213>  unknown

<220>
<223>  Q5VZ03 - thioredoxin domain containing protein C9orf121

<400>  47

Met Val Asp Ile Leu Gly Glu Arg His Leu Val Thr Cys Lys Gly Ala
1               5                   10                  15

Thr Val Glu Ala Glu Ala Ala Leu Gln Asn Lys Val Val Ala Leu Tyr
            20                  25                  30

Phe Ala Ala Ala Arg Cys Ala Pro Ser Arg Asp Phe Thr Pro Leu Leu
        35                  40                  45

Cys Asp Phe Tyr Thr Ala Leu Val Ala Glu Ala Arg Arg Pro Ala Pro
    50                  55                  60

Phe Glu Val Val Phe Val Ser Ala Asp Gly Ser Ser Gln Glu Met Leu
65                  70                  75                  80

Asp Phe Met Arg Glu Leu His Gly Ala Trp Leu Ala Leu Pro Phe His
                85                  90                  95

Asp Pro Tyr Arg His Glu Leu Arg Lys Arg Tyr Asn Val Thr Ala Ile
            100                 105                 110

Pro Lys Leu Val Ile Val Lys Gln Asn Gly Glu Val Ile Thr Asn Lys
        115                 120                 125

Gly Arg Lys Gln Ile Arg Glu Arg Gly Leu Ala Cys Phe Gln Asp Trp
    130                 135                 140

Val Glu Ala Ala Asp Ile Phe Gln Asn Phe Ser Val
145                 150                 155

<210>  48
<211>  156
<212>  PRT
<213>  unknown

<220>
<223>  P62688

<400>  48

Met Gly Gln Thr Lys Ser Lys Ile Lys Ser Lys Tyr Ala Ser His Leu
1               5                   10                  15

Ser Phe Ile Lys Ile Leu Leu Lys Arg Gly Gly Val Lys Val Ser Thr
            20                  25                  30

Lys Asn Leu Ile Lys Leu Phe Gln Ile Ile Glu Gln Phe Cys Pro Trp
        35                  40                  45

Phe Pro Glu Gln Gly Thr Leu Asp Leu Lys Asp Trp Lys Arg Ile Gly
    50                  55                  60

Lys Glu Leu Lys Gln Ala Gly Arg Lys Gly Asn Ile Ile Pro Leu Thr
65                  70                  75                  80

Val Trp Asn Asp Trp Ala Ile Ile Lys Ala Ala Leu Glu Pro Phe Gln
                85                  90                  95

Thr Glu Glu Asp Ser Val Ser Val Ser Asp Ala Pro Gly Ser Cys Leu
            100                 105                 110

Ile Asp Cys Asn Glu Lys Thr Arg Lys Lys Ser Gln Lys Glu Thr Glu
        115                 120                 125

Ser Leu His Cys Glu Tyr Val Ala Glu Pro Val Met Ala Gln Ser Thr
    130                 135                 140

Gln Asn Val Asp Tyr Asn Gln Leu Gln Glu Val Ile
145                 150                 155

<210>  49
<211>  159
<212>  PRT
<213>  unknown

<220>
<223>  P47914 - 60S ribosomal protein L29

<400>  49

<210>  50
<211>  156
<212>  PRT
<213>  unknown

<220>
<223>  Q8N2H4 - protein SYS1 homolog

<400>  50

Met Ala Gly Gln Phe Arg Ser Tyr Val Trp Asp Pro Leu Leu Ile Leu
1               5                   10                  15

Ser Gln Ile Val Leu Met Gln Thr Val Tyr Tyr Gly Ser Leu Gly Leu
            20                  25                  30

Trp Leu Ala Leu Val Asp Gly Leu Val Arg Ser Ser Pro Ser Leu Asp
        35                  40                  45

Gln Met Phe Asp Ala Glu Ile Leu Gly Phe Ser Thr Pro Pro Gly Arg
    50                  55                  60

Leu Ser Met Met Ser Phe Ile Leu Asn Ala Leu Thr Cys Ala Leu Gly
65                  70                  75                  80

Leu Leu Tyr Phe Ile Arg Arg Gly Lys Gln Cys Leu Asp Phe Thr Val
                85                  90                  95

Thr Val His Phe Phe His Leu Leu Gly Cys Trp Phe Tyr Ser Ser Arg
            100                 105                 110

Phe Pro Ser Ala Leu Thr Trp Trp Leu Val Gln Ala Val Cys Ile Ala
        115                 120                 125

Leu Met Ala Val Ile Gly Glu Tyr Leu Cys Met Arg Thr Glu Leu Lys
    130                 135                 140

Glu Ile Pro Leu Asn Ser Ala Pro Lys Ser Asn Val
145                 150                 155


<210>  51
<211>  155
<212>  PRT
<213>  unknown

<220>
<223>  Q15649 - zinc finger HIT domain containing protein 3

<400>  51

<210>  52
<211>  318
<212>  PRT
<213>  unknown

<220>
<223>  Q10588 - ADP ribosyl cyclase 2

<400>  52

Met Ala Ala Gln Gly Cys Ala Ala Ser Arg Leu Leu Gln Leu Leu Leu
1               5                   10                  15

Gln Leu Leu Leu Leu Leu Leu Leu Leu Ala Ala Gly Gly Ala Arg Ala
            20                  25                  30

Arg Trp Arg Gly Glu Gly Thr Ser Ala His Leu Arg Asp Ile Phe Leu
        35                  40                  45

Gly Arg Cys Ala Glu Tyr Arg Ala Leu Leu Ser Pro Glu Gln Arg Asn
    50                  55                  60

Lys Asn Cys Thr Ala Ile Trp Glu Ala Phe Lys Val Ala Leu Asp Lys
65                  70                  75                  80

Asp Pro Cys Ser Val Leu Pro Ser Asp Tyr Asp Leu Phe Ile Asn Leu
                85                  90                  95

Ser Arg His Ser Ile Pro Arg Asp Lys Ser Leu Phe Trp Glu Asn Ser
            100                 105                 110

His Leu Leu Val Asn Ser Phe Ala Asp Asn Thr Arg Arg Phe Met Pro
        115                 120                 125

Leu Ser Asp Val Leu Tyr Gly Arg Val Ala Asp Phe Leu Ser Trp Cys

130                 135                 140

Arg Gln Lys Asn Asp Ser Gly Leu Asp Tyr Gln Ser Cys Pro Thr Ser
145                 150                 155                 160

Glu Asp Cys Glu Asn Asn Pro Val Asp Ser Phe Trp Lys Arg Ala Ser
                165                 170                 175

Ile Gln Tyr Ser Lys Asp Ser Ser Gly Val Ile His Val Met Leu Asn
            180                 185                 190

Gly Ser Glu Pro Thr Gly Ala Tyr Pro Ile Lys Gly Phe Phe Ala Asp
        195                 200                 205

Tyr Glu Ile Pro Asn Leu Gln Lys Glu Lys Ile Thr Arg Ile Glu Ile
    210                 215                 220

Trp Val Met His Glu Ile Gly Gly Pro Asn Val Glu Ser Cys Gly Glu
225                 230                 235                 240

Gly Ser Met Lys Val Leu Glu Lys Arg Leu Lys Asp Met Gly Phe Gln
                245                 250                 255

Tyr Ser Cys Ile Asn Asp Tyr Arg Pro Val Lys Leu Leu Gln Cys Val
            260                 265                 270

Asp His Ser Thr His Pro Asp Cys Ala Leu Lys Ser Ala Ala Ala Ala
        275                 280                 285

Thr Gln Arg Lys Ala Pro Ser Leu Tyr Thr Glu Gln Arg Ala Gly Leu
    290                 295                 300

Ile Ile Pro Leu Phe Leu Val Leu Ala Ser Arg Thr Gln Leu
305                 310                 315

<210>  53
<211>  255
<212>  PRT
<213>  unknown

<220>
<223>  Q8TCB7 - methyltransferase like protein 6

<400>  53

Met Ala Ser Leu Gln Arg Lys Gly Leu Gln Ala Arg Ile Leu Thr Ser
1               5                   10                  15

Glu Glu Glu Glu Lys Leu Lys Arg Asp Gln Thr Leu Val Ser Asp Phe
            20                  25                  30

Lys Gln Gln Lys Leu Glu Gln Glu Ala Gln Lys Asn Trp Asp Leu Phe
        35                  40                  45

Tyr Lys Arg Asn Ser Thr Asn Phe Phe Lys Asp Arg His Trp Thr Thr
    50                  55                  60

Arg Glu Phe Glu Glu Leu Arg Ser Cys Arg Glu Phe Glu Asp Gln Lys
65                  70                  75                  80

Leu Thr Met Leu Glu Ala Gly Cys Gly Val Gly Asn Cys Leu Phe Pro
                85                  90                  95

Leu Leu Glu Glu Asp Pro Asn Ile Phe Ala Tyr Ala Cys Asp Phe Ser
            100                 105                 110

Pro Arg Ala Ile Glu Tyr Val Lys Gln Asn Pro Leu Tyr Asp Thr Glu
        115                 120                 125

Arg Cys Lys Val Phe Gln Cys Asp Leu Thr Lys Asp Asp Leu Leu Asp
    130                 135                 140

His Val Pro Pro Glu Ser Val Asp Val Val Met Leu Ile Phe Val Leu
145                 150                 155                 160

Ser Ala Val His Pro Asp Lys Met His Leu Val Leu Gln Asn Ile Tyr
                165                 170                 175

Lys Val Leu Lys Pro Gly Lys Ser Val Leu Phe Arg Asp Tyr Gly Leu
            180                 185                 190

Tyr Asp His Ala Met Leu Arg Phe Lys Ala Ser Ser Lys Leu Gly Glu
        195                 200                 205

Asn Phe Tyr Val Arg Gln Asp Gly Thr Arg Ser Tyr Phe Phe Thr Asp
    210                 215                 220

Asp Phe Leu Ala Gln Leu Phe Met Asp Thr Gly Tyr Glu Glu Val Val
225                 230                 235                 240

Asn Glu Tyr Val Phe Arg Glu Thr Val Asn Lys Lys Glu Gly Leu
                245                 250                 255

<210>  54
<211>  274
<212>  PRT
<213>  unknown

<220>
<223>  Q8NA92 - THAP domain containing protein 8

<400>  54

Met Pro Lys Tyr Cys Arg Ala Pro Asn Cys Ser Asn Thr Ala Gly Arg
1               5                   10                  15

Leu Gly Ala Asp Asn Arg Pro Val Ser Phe Tyr Lys Phe Pro Leu Lys
            20                  25                  30

Asp Gly Pro Arg Leu Gln Ala Trp Leu Gln His Met Gly Cys Glu His
        35                  40                  45

Trp Val Pro Ser Cys His Gln His Leu Cys Ser Glu His Phe Thr Pro
    50                  55                  60

Ser Cys Phe Gln Trp Arg Trp Gly Val Arg Tyr Leu Arg Pro Asp Ala
65                  70                  75                  80

Val Pro Ser Ile Phe Ser Arg Gly Pro Pro Ala Lys Ser Gln Arg Arg
                85                  90                  95

Thr Arg Ser Thr Gln Lys Pro Val Ser Pro Pro Pro Pro Leu Gln Lys
            100                 105                 110

Asn Thr Pro Leu Pro Gln Ser Pro Ala Ile Pro Val Ser Gly Pro Val
        115                 120                 125

Arg Leu Val Val Leu Gly Pro Thr Ser Gly Ser Pro Lys Thr Val Ala
    130                 135                 140

Thr Met Leu Leu Thr Pro Leu Ala Pro Ala Pro Thr Pro Glu Arg Ser
145                 150                 155                 160

Gln Pro Glu Val Pro Ala Gln Gln Ala Gln Thr Gly Leu Gly Pro Val
                165                 170                 175

Leu Gly Ala Leu Gln Arg Arg Val Arg Arg Leu Gln Arg Cys Gln Glu
            180                 185                 190

Arg His Gln Ala Gln Leu Gln Ala Leu Glu Arg Leu Ala Gln Gln Leu
        195                 200                 205

His Gly Glu Ser Leu Leu Ala Arg Ala Arg Arg Gly Leu Gln Arg Leu
    210                 215                 220

Thr Thr Ala Gln Thr Leu Gly Pro Glu Glu Ser Gln Thr Phe Thr Ile
225                 230                 235                 240

Ile Cys Gly Gly Pro Asp Ile Ala Met Val Leu Ala Gln Asp Pro Ala
                245                 250                 255

Pro Ala Thr Val Asp Ala Lys Pro Glu Leu Leu Asp Thr Arg Ile Pro
            260                 265                 270

Ser Ala

<210>  55
<211>  318
<212>  PRT
<213>  unknown

<220>
<223>  O75208 - ubiquinone biosynthesis protein COQ9

<400>  55

Met Ala Ala Ala Ala Val Ser Gly Ala Leu Gly Arg Ala Gly Trp Arg
1               5                   10                  15

Leu Leu Gln Leu Arg Cys Leu Pro Val Ala Arg Cys Arg Gln Ala Leu
            20                  25                  30

Val Pro Arg Ala Phe His Ala Ser Ala Val Gly Leu Arg Ser Ser Asp
        35                  40                  45

Glu Gln Lys Gln Gln Pro Pro Asn Ser Phe Ser Gln Gln His Ser Glu
    50                  55                  60

Thr Gln Gly Ala Glu Lys Pro Asp Pro Glu Ser Ser His Ser Pro Pro
65                  70                  75                  80

Arg Tyr Thr Asp Gln Gly Gly Glu Glu Glu Glu Asp Tyr Glu Ser Glu
                85                  90                  95

Glu Gln Leu Gln His Arg Ile Leu Thr Ala Ala Leu Glu Phe Val Pro
            100                 105                 110

Ala His Gly Trp Thr Ala Glu Ala Ile Ala Glu Gly Ala Gln Ser Leu
        115                 120                 125

Gly Leu Ser Ser Ala Ala Ala Ser Met Phe Gly Lys Asp Gly Ser Glu
    130                 135                 140

Leu Ile Leu His Phe Val Thr Gln Cys Asn Thr Arg Leu Thr Arg Val
145                 150                 155                 160

Leu Glu Glu Glu Gln Lys Leu Val Gln Leu Gly Gln Ala Glu Lys Arg
                165                 170                 175

Lys Thr Asp Gln Phe Leu Arg Asp Ala Val Glu Thr Arg Leu Arg Met
            180                 185                 190

Leu Ile Pro Tyr Ile Glu His Trp Pro Arg Ala Leu Ser Ile Leu Met
        195                 200                 205

Leu Pro His Asn Ile Pro Ser Ser Leu Ser Leu Leu Thr Ser Met Val
    210                 215                 220

Asp Asp Met Trp His Tyr Ala Gly Asp Gln Ser Thr Asp Phe Asn Trp
225                 230                 235                 240

Tyr Thr Arg Arg Ala Met Leu Ala Ala Ile Tyr Asn Thr Thr Glu Leu
                245                 250                 255

Val Met Met Gln Asp Ser Ser Pro Asp Phe Glu Asp Thr Trp Arg Phe
            260                 265                 270

Leu Glu Asn Arg Val Asn Asp Ala Met Asn Met Gly His Thr Ala Lys
        275                 280                 285

Gln Val Lys Ser Thr Gly Glu Ala Leu Val Gln Gly Leu Met Gly Ala
    290                 295                 300

Ala Val Thr Leu Lys Asn Leu Thr Gly Leu Asn Gln Arg Arg
305                 310                 315

<210>  56
<211>  297
<212>  PRT
<213>  unknown

<220>
<223>  P28360 - homeobox portein MSX-1

<400>  56

Met Thr Ser Leu Pro Leu Gly Val Lys Val Glu Asp Ser Ala Phe Gly
1               5                   10                  15

Lys Pro Ala Gly Gly Gly Ala Gly Gln Ala Pro Ser Ala Ala Ala Ala
            20                  25                  30

Thr Ala Ala Ala Met Gly Ala Asp Glu Glu Gly Ala Lys Pro Lys Val
        35                  40                  45

Ser Pro Ser Leu Leu Pro Phe Ser Val Glu Ala Leu Met Ala Asp His
    50                  55                  60

Arg Lys Pro Gly Ala Lys Glu Ser Ala Leu Ala Pro Ser Glu Gly Val
65                  70                  75                  80

Gln Ala Ala Gly Gly Ser Ala Gln Pro Leu Gly Val Pro Pro Gly Ser
                85                  90                  95

Leu Gly Ala Pro Asp Ala Pro Ser Ser Pro Arg Pro Leu Gly His Phe
            100                 105                 110

Ser Val Gly Gly Leu Leu Lys Leu Pro Glu Asp Ala Leu Val Lys Ala
        115                 120                 125

Glu Ser Pro Glu Lys Pro Glu Arg Thr Pro Trp Met Gln Ser Pro Arg
    130                 135                 140

Phe Ser Pro Pro Pro Ala Arg Arg Leu Ser Pro Pro Ala Cys Thr Leu
145                 150                 155                 160

Arg Lys His Lys Thr Asn Arg Lys Pro Arg Thr Pro Phe Thr Thr Ala
                165                 170                 175

Gln Leu Leu Ala Leu Glu Arg Lys Phe Arg Gln Lys Gln Tyr Leu Ser
            180                 185                 190

Ile Ala Glu Arg Ala Glu Phe Ser Ser Ser Leu Ser Leu Thr Glu Thr
        195                 200                 205

Gln Val Lys Ile Trp Phe Gln Asn Arg Arg Ala Lys Ala Lys Arg Leu
    210                 215                 220

Gln Glu Ala Glu Leu Glu Lys Leu Lys Met Ala Ala Lys Pro Met Leu
225                 230                 235                 240

Pro Pro Ala Ala Phe Gly Leu Ser Phe Pro Leu Gly Gly Pro Ala Ala
                245                 250                 255

Val Ala Ala Ala Ala Gly Ala Ser Leu Tyr Gly Ala Ser Gly Pro Phe
            260                 265                 270

Gln Arg Ala Ala Leu Pro Val Ala Pro Val Gly Leu Tyr Thr Ala His
        275                 280                 285

Val Gly Tyr Ser Met Tyr His Leu Thr
    290                 295


<210>  57
<211>  273
<212>  PRT
<213>  unknown

<220>
<223>  Q96BD6 - SOCS box protein

<400>  57

Met Gly Gln Lys Val Thr Gly Gly Ile Lys Thr Val Asp Met Arg Asp
1               5                   10                  15

Pro Thr Tyr Arg Pro Leu Lys Gln Glu Leu Gln Gly Leu Asp Tyr Cys
            20                  25                  30

Lys Pro Thr Arg Leu Asp Leu Leu Leu Asp Met Pro Pro Val Ser Tyr
        35                  40                  45

Asp Val Gln Leu Leu His Ser Trp Asn Asn Asn Asp Arg Ser Leu Asn
    50                  55                  60

Val Phe Val Lys Glu Asp Asp Lys Leu Ile Phe His Arg His Pro Val
65                  70                  75                  80

Ala Gln Ser Thr Asp Ala Ile Arg Gly Lys Val Gly Tyr Thr Arg Gly
                85                  90                  95

Leu His Val Trp Gln Ile Thr Trp Ala Met Arg Gln Arg Gly Thr His

            100                 105                 110

Ala Val Val Gly Val Ala Thr Ala Asp Ala Pro Leu His Ser Val Gly
        115                 120                 125

Tyr Thr Thr Leu Val Gly Asn Asn His Glu Ser Trp Gly Trp Asp Leu
    130                 135                 140

Gly Arg Asn Arg Leu Tyr His Asp Gly Lys Asn Gln Pro Ser Lys Thr
145                 150                 155                 160

Tyr Pro Ala Phe Leu Glu Pro Asp Glu Thr Phe Ile Val Pro Asp Ser
                165                 170                 175

Phe Leu Val Ala Leu Asp Met Asp Asp Gly Thr Leu Ser Phe Ile Val
            180                 185                 190

Asp Gly Gln Tyr Met Gly Val Ala Phe Arg Gly Leu Lys Gly Lys Lys
        195                 200                 205

Leu Tyr Pro Val Val Ser Ala Val Trp Gly His Cys Glu Ile Arg Met
    210                 215                 220

Arg Tyr Leu Asn Gly Leu Asp Pro Glu Pro Leu Pro Leu Met Asp Leu
225                 230                 235                 240

Cys Arg Arg Ser Val Arg Leu Ala Leu Gly Arg Glu Arg Leu Gly Glu
                245                 250                 255

Ile His Thr Leu Pro Leu Pro Ala Ser Leu Lys Ala Tyr Leu Leu Tyr
            260                 265                 270

Gln

<210> 58
<211> 562
<212> PRT
<213> unknown

<220>
<223> P00750 - tisue type plasminogen activator

<400> 58

Met Asp Ala Met Lys Arg Gly Leu Cys Cys Val Leu Leu Leu Cys Gly
1               5                   10                  15

Ala Val Phe Val Ser Pro Ser Gln Glu Ile His Ala Arg Phe Arg Arg
            20                  25                  30

Gly Ala Arg Ser Tyr Gln Val Ile Cys Arg Asp Glu Lys Thr Gln Met
        35                  40                  45

Ile Tyr Gln Gln His Gln Ser Trp Leu Arg Pro Val Leu Arg Ser Asn
    50                  55                  60

Arg Val Glu Tyr Cys Trp Cys Asn Ser Gly Arg Ala Gln Cys His Ser
65                  70                  75                  80

Val Pro Val Lys Ser Cys Ser Glu Pro Arg Cys Phe Asn Gly Gly Thr
                85                  90                  95

Cys Gln Gln Ala Leu Tyr Phe Ser Asp Phe Val Cys Gln Cys Pro Glu
            100                 105                 110

Gly Phe Ala Gly Lys Cys Cys Glu Ile Asp Thr Arg Ala Thr Cys Tyr
        115                 120                 125

Glu Asp Gln Gly Ile Ser Tyr Arg Gly Thr Trp Ser Thr Ala Glu Ser
    130                 135                 140

Gly Ala Glu Cys Thr Asn Trp Asn Ser Ser Ala Leu Ala Gln Lys Pro
145                 150                 155                 160

Tyr Ser Gly Arg Arg Pro Asp Ala Ile Arg Leu Gly Leu Gly Asn His
                165                 170                 175

Asn Tyr Cys Arg Asn Pro Asp Arg Asp Ser Lys Pro Trp Cys Tyr Val
            180                 185                 190

Phe Lys Ala Gly Lys Tyr Ser Ser Glu Phe Cys Ser Thr Pro Ala Cys
        195                 200                 205

Ser Glu Gly Asn Ser Asp Cys Tyr Phe Gly Asn Gly Ser Ala Tyr Arg
    210                 215                 220

Gly Thr His Ser Leu Thr Glu Ser Gly Ala Ser Cys Leu Pro Trp Asn
225                 230                 235                 240

Ser Met Ile Leu Ile Gly Lys Val Tyr Thr Ala Gln Asn Pro Ser Ala
                245                 250                 255

Gln Ala Leu Gly Leu Gly Lys His Asn Tyr Cys Arg Asn Pro Asp Gly
            260                 265                 270

Asp Ala Lys Pro Trp Cys His Val Leu Lys Asn Arg Arg Leu Thr Trp
        275                 280                 285

Glu Tyr Cys Asp Val Pro Ser Cys Ser Thr Cys Gly Leu Arg Gln Tyr
    290                 295                 300

Ser Gln Pro Gln Phe Arg Ile Lys Gly Gly Leu Phe Ala Asp Ile Ala
305                 310                 315                 320

Ser His Pro Trp Gln Ala Ala Ile Phe Ala Lys His Arg Arg Ser Pro

                325                 330                 335

Gly Glu Arg Phe Leu Cys Gly Gly Ile Leu Ile Ser Ser Cys Trp Ile
            340                 345                 350

Leu Ser Ala Ala His Cys Phe Gln Glu Arg Phe Pro Pro His His Leu
        355                 360                 365

Thr Val Ile Leu Gly Arg Thr Tyr Arg Val Val Pro Gly Glu Glu Glu
    370                 375                 380

Gln Lys Phe Glu Val Glu Lys Tyr Ile Val His Lys Glu Phe Asp Asp
385                 390                 395                 400

Asp Thr Tyr Asp Asn Asp Ile Ala Leu Leu Gln Leu Lys Ser Asp Ser
                405                 410                 415

Ser Arg Cys Ala Gln Glu Ser Ser Val Val Arg Thr Val Cys Leu Pro
            420                 425                 430

Pro Ala Asp Leu Gln Leu Pro Asp Trp Thr Glu Cys Glu Leu Ser Gly
        435                 440                 445

Tyr Gly Lys His Glu Ala Leu Ser Pro Phe Tyr Ser Glu Arg Leu Lys
    450                 455                 460

Glu Ala His Val Arg Leu Tyr Pro Ser Ser Arg Cys Thr Ser Gln His
465                 470                 475                 480

Leu Leu Asn Arg Thr Val Thr Asp Asn Met Leu Cys Ala Gly Asp Thr
                485                 490                 495

Arg Ser Gly Gly Pro Gln Ala Asn Leu His Asp Ala Cys Gln Gly Asp
            500                 505                 510

Ser Gly Gly Pro Leu Val Cys Leu Asn Asp Gly Arg Met Thr Leu Val
        515                 520                 525

Gly Ile Ile Ser Trp Gly Leu Gly Cys Gly Gln Lys Asp Val Pro Gly
    530                 535                 540

Val Tyr Thr Lys Val Thr Asn Tyr Leu Asp Trp Ile Arg Asp Asn Met
545                 550                 555                 560

Arg Pro

<210> 59
<211> 313
<212> PRT
<213> unknown

<220>

<223>  Q9Y6I9 - 264 protein

<400>  59

Met Ser Asp Leu Leu Leu Leu Gly Leu Ile Gly Gly Leu Thr Leu Leu
1               5                   10                  15

Leu Leu Leu Thr Leu Leu Ala Phe Ala Gly Tyr Ser Gly Leu Leu Ala
            20                  25                  30

Gly Val Glu Val Ser Ala Gly Ser Pro Pro Ile Arg Asn Val Thr Val
        35                  40                  45

Ala Tyr Lys Phe His Met Gly Leu Tyr Gly Glu Thr Gly Arg Leu Phe
    50                  55                  60

Thr Glu Ser Cys Ser Ile Ser Pro Lys Leu Arg Ser Ile Ala Val Tyr
65                  70                  75                  80

Tyr Asp Asn Pro His Met Val Pro Pro Asp Lys Cys Arg Cys Ala Val
                85                  90                  95

Gly Ser Ile Leu Ser Glu Gly Glu Glu Ser Pro Ser Pro Glu Leu Ile
            100                 105                 110

Asp Leu Tyr Gln Lys Phe Gly Phe Lys Val Phe Ser Phe Pro Ala Pro
        115                 120                 125

Ser His Val Val Thr Ala Thr Phe Pro Tyr Thr Thr Ile Leu Ser Ile
    130                 135                 140

Trp Leu Ala Thr Arg Arg Val His Pro Ala Leu Asp Thr Tyr Ile Lys
145                 150                 155                 160

Glu Arg Lys Leu Cys Ala Tyr Pro Arg Leu Glu Ile Tyr Gln Glu Asp
                165                 170                 175

Gln Ile His Phe Met Cys Pro Leu Ala Arg Gln Gly Asp Phe Tyr Val
            180                 185                 190

Pro Glu Met Lys Glu Thr Glu Trp Lys Trp Arg Gly Leu Val Glu Ala
        195                 200                 205

Ile Asp Thr Gln Val Asp Gly Thr Gly Ala Asp Thr Met Ser Asp Thr
    210                 215                 220

Ser Ser Val Ser Leu Glu Val Ser Pro Gly Ser Arg Glu Thr Ser Ala
225                 230                 235                 240

Ala Thr Leu Ser Pro Gly Ala Ser Ser Arg Gly Trp Asp Asp Gly Asp
                245                 250                 255

Thr Arg Ser Glu His Ser Tyr Ser Glu Ser Gly Ala Ser Gly Ser Ser

260                 265                 270

Phe Glu Glu Leu Asp Leu Glu Gly Glu Gly Pro Leu Gly Glu Ser Arg
        275                 280                 285

Leu Asp Pro Gly Thr Glu Pro Leu Gly Thr Thr Lys Trp Leu Trp Glu
    290                 295                 300

Pro Thr Ala Pro Glu Lys Gly Lys Glu
305                 310

<210>  60
<211>  290
<212>  PRT
<213>  unknown

<220>
<223>  Q14135 - TF cofactor vestigial-like protein 4

<400>  60

Met Glu Thr Pro Leu Asp Val Leu Ser Arg Ala Ala Ser Leu Val His
1               5                   10                  15

Ala Asp Asp Glu Lys Arg Glu Ala Ala Leu Arg Gly Glu Pro Arg Met
            20                  25                  30

Gln Thr Leu Pro Val Ala Ser Ala Leu Ser Ser His Arg Thr Gly Pro
        35                  40                  45

Pro Pro Ile Ser Pro Ser Lys Arg Lys Phe Ser Met Glu Pro Gly Asp
    50                  55                  60

Glu Asp Leu Asp Cys Asp Asn Asp His Val Ser Lys Met Ser Arg Ile
65                  70                  75                  80

Phe Asn Pro His Leu Asn Lys Thr Ala Asn Gly Asp Cys Arg Arg Asp
                85                  90                  95

Pro Arg Glu Arg Ser Arg Ser Pro Ile Glu Arg Ala Val Ala Pro Thr
            100                 105                 110

Met Ser Leu His Gly Ser His Leu Tyr Thr Ser Leu Pro Ser Leu Gly
        115                 120                 125

Leu Glu Gln Pro Leu Ala Leu Thr Lys Asn Ser Leu Asp Ala Ser Arg
    130                 135                 140

Pro Ala Gly Leu Ser Pro Thr Leu Thr Pro Gly Glu Arg Gln Gln Asn
145                 150                 155                 160

Arg Pro Ser Val Ile Thr Cys Ala Ser Ala Gly Ala Arg Asn Cys Asn
                165                 170                 175

Leu Ser His Cys Pro Ile Ala His Ser Gly Cys Ala Ala Pro Gly Pro
            180                 185                 190

Ala Ser Tyr Arg Arg Pro Pro Ser Ala Ala Thr Thr Cys Asp Pro Val
        195                 200                 205

Val Glu Glu His Phe Arg Arg Ser Leu Gly Lys Asn Tyr Lys Glu Pro
    210                 215                 220

Glu Pro Ala Pro Asn Ser Val Ser Ile Thr Gly Ser Val Asp Asp His
225                 230                 235                 240

Phe Ala Lys Ala Leu Gly Asp Thr Trp Leu Gln Ile Lys Ala Ala Lys
                245                 250                 255

Asp Gly Ala Ser Ser Ser Pro Glu Ser Ala Ser Arg Arg Gly Gln Pro
            260                 265                 270

Ala Ser Pro Ser Ala His Met Val Ser His Ser His Ser Pro Ser Val
        275                 280                 285

Val Ser
    290

<210>  61
<211>  285
<212>  PRT
<213>  unknown

<220>
<223>  O94760 - NG,NG dimethylarginine dimethylaminohydrolase 1

<400>  61

Met Ala Gly Leu Gly His Pro Ala Ala Phe Gly Arg Ala Thr His Ala
1               5                   10                  15

Val Val Arg Ala Leu Pro Glu Ser Leu Gly Gln His Ala Leu Arg Ser
            20                  25                  30

Ala Lys Gly Glu Glu Val Asp Val Ala Arg Ala Glu Arg Gln His Gln
        35                  40                  45

Leu Tyr Val Gly Val Leu Gly Ser Lys Leu Gly Leu Gln Val Val Glu
    50                  55                  60

Leu Pro Ala Asp Glu Ser Leu Pro Asp Cys Val Phe Val Glu Asp Val
65                  70                  75                  80

Ala Val Val Cys Glu Glu Thr Ala Leu Ile Thr Arg Pro Gly Ala Pro
                85                  90                  95

Ser Arg Arg Lys Glu Val Asp Met Met Lys Glu Ala Leu Glu Lys Leu
            100                 105                 110

Gln Leu Asn Ile Val Glu Met Lys Asp Glu Asn Ala Thr Leu Asp Gly
        115                 120                 125

Gly Asp Val Leu Phe Thr Gly Arg Glu Phe Phe Val Gly Leu Ser Lys
    130                 135                 140

Arg Thr Asn Gln Arg Gly Ala Glu Ile Leu Ala Asp Thr Phe Lys Asp
145                 150                 155                 160

Tyr Ala Val Ser Thr Val Pro Val Ala Asp Gly Leu His Leu Lys Ser
                165                 170                 175

Phe Cys Ser Met Ala Gly Pro Asn Leu Ile Ala Ile Gly Ser Ser Glu
            180                 185                 190

Ser Ala Gln Lys Ala Leu Lys Ile Met Gln Gln Met Ser Asp His Arg
        195                 200                 205

Tyr Asp Lys Leu Thr Val Pro Asp Asp Ile Ala Ala Asn Cys Ile Tyr
    210                 215                 220

Leu Asn Ile Pro Asn Lys Gly His Val Leu Leu His Arg Thr Pro Glu
225                 230                 235                 240

Glu Tyr Pro Glu Ser Ala Lys Val Tyr Glu Lys Leu Lys Asp His Met
                245                 250                 255

Leu Ile Pro Val Ser Met Ser Glu Leu Glu Lys Val Asp Gly Leu Leu
            260                 265                 270

Thr Cys Cys Ser Val Leu Ile Asn Lys Lys Val Asp Ser
        275                 280                 285

<210>  62
<211>  281
<212>  PRT
<213>  unknown

<220>
<223>  P29692 - elaongation factor 1-delta

<400>  62

Met Ala Thr Asn Phe Leu Ala His Glu Lys Ile Trp Phe Asp Lys Phe
1               5                   10                  15

Lys Tyr Asp Asp Ala Glu Arg Arg Phe Tyr Glu Gln Met Asn Gly Pro
            20                  25                  30

Val Ala Gly Ala Ser Arg Gln Glu Asn Gly Ala Ser Val Ile Leu Arg
        35                  40                  45

Asp Ile Ala Arg Ala Arg Glu Asn Ile Gln Lys Ser Leu Ala Gly Ser
    50                  55                  60

Ser Gly Pro Gly Ala Ser Ser Gly Thr Ser Gly Asp His Gly Glu Leu
65                  70                  75                  80

Val Val Arg Ile Ala Ser Leu Glu Val Glu Asn Gln Ser Leu Arg Gly
                85                  90                  95

Val Val Gln Glu Leu Gln Gln Ala Ile Ser Lys Leu Glu Ala Arg Leu
            100                 105                 110

Asn Val Leu Glu Lys Ser Ser Pro Gly His Arg Ala Thr Ala Pro Gln
        115                 120                 125

Thr Gln His Val Ser Pro Met Arg Gln Val Glu Pro Pro Ala Lys Lys
    130                 135                 140

Pro Ala Thr Pro Ala Glu Asp Asp Glu Asp Asp Asp Ile Asp Leu Phe
145                 150                 155                 160

Gly Ser Asp Asn Glu Glu Glu Asp Lys Glu Ala Ala Gln Leu Arg Glu
                165                 170                 175

Glu Arg Leu Arg Gln Tyr Ala Glu Lys Lys Ala Lys Lys Pro Ala Leu
            180                 185                 190

Val Ala Lys Ser Ser Ile Leu Leu Asp Val Lys Pro Trp Asp Asp Glu
        195                 200                 205

Thr Asp Met Ala Gln Leu Glu Ala Cys Val Arg Ser Ile Gln Leu Asp
    210                 215                 220

Gly Leu Val Trp Gly Ala Ser Lys Leu Val Pro Val Gly Tyr Gly Ile
225                 230                 235                 240

Arg Lys Leu Gln Ile Gln Cys Val Val Glu Asp Asp Lys Val Gly Thr
                245                 250                 255

Asp Leu Leu Glu Glu Glu Ile Thr Lys Phe Glu Glu His Val Gln Ser
            260                 265                 270

Val Asp Ile Ala Ala Phe Asn Lys Ile
        275                 280

<210>  63
<211>  21
<212>  PRT
<213>  unknown

<220>
<223>  insulin alpha chain

<400>  63

Gly Ile Val Glu Gln Cys Cys Thr Ser Ile Cys Ser Leu Tyr Gln Leu

1               5                   10                  15

Glu Asn Tyr Cys Asn
            20

<210>  64
<211>  30
<212>  PRT
<213>  unknown

<220>
<223>  insulin beta chain

<400>  64

Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr
            20                  25                  30

<210>  65
<211>  26
<212>  PRT
<213>  unknown

<220>
<223>  insulin like 3A chain

<400>  65

Ala Ala Ala Thr Asn Pro Ala Arg Tyr Cys Cys Leu Ser Gly Cys Thr
1               5                   10                  15

Gln Gln Asp Leu Leu Thr Leu Cys Pro Tyr
            20                  25

<210>  66
<211>  14
<212>  PRT
<213>  unknown

<220>
<223>  somatostatin 14

<400>  66

Ala Gly Cys Lys Asn Phe Phe Trp Lys Thr Phe Thr Ser Cys
1               5                   10

<210>  67
<211>  28
<212>  PRT
<213>  unknown

<220>
<223>  somatostatin 28

<400>  67

Ser Ala Asn Ser Asn Pro Ala Met Ala Pro Arg Glu Arg Lys Ala Gly
1               5                   10                  15

Cys Lys Asn Phe Phe Trp Lys Thr Phe Thr Ser Cys
            20                  25

<210>  68
<211>  6
<212>  PRT
<213>  unknown

<220>
<223>  gastrin-6

<400>  68

Tyr Gly Trp Met Asp Phe
1               5

<210>  69
<211>  14
<212>  PRT
<213>  unknown

<220>
<223>  gastrin-14

<400>  69

Trp Leu Glu Glu Glu Glu Glu Ala Tyr Gly Trp Met Asp Phe
1               5                   10

<210>  70
<211>  34
<212>  PRT
<213>  unknown

<220>
<223>  gastrin-34

<400>  70

Gln Leu Gly Pro Gln Gly Pro Pro His Leu Val Ala Asp Pro Ser Lys
1               5                   10                  15

Lys Gln Gly Pro Trp Leu Glu Glu Glu Glu Glu Ala Tyr Gly Trp Met
            20                  25                  30

Asp Phe

<210>  71
<211>  52
<212>  PRT
<213>  unknown

<220>
<223>  gastrin 52

<400>  71

Asp Leu Glu Leu Pro Trp Leu Glu Gln Gln Gly Pro Ala Ser His His
1               5                   10                  15

Arg Arg Gln Leu Gly Pro Gln Gly Pro Pro His Leu Val Ala Asp Pro
            20                  25                  30

Ser Lys Lys Gln Gly Pro Trp Leu Glu Glu Glu Glu Glu Ala Tyr Gly
        35                  40                  45

Trp Met Asp Phe
    50

<210>  72
<211>  71
<212>  PRT
<213>  unknown

<220>
<223>  gastrin-71

<400>  72

Ser Trp Lys Pro Arg Ser Gln Gln Pro Asp Ala Pro Leu Gly Thr Gly
1               5                   10                  15

Ala Asn Arg Asp Leu Glu Leu Pro Trp Leu Glu Gln Gln Gly Pro Ala
            20                  25                  30

Ser His His Arg Arg Gln Leu Gly Pro Gln Gly Pro Pro His Leu Val
        35                  40                  45

Ala Asp Pro Ser Lys Lys Gln Gly Pro Trp Leu Glu Glu Glu Glu Glu
    50                  55                  60

Ala Tyr Gly Trp Met Asp Phe
65                  70

<210>  73
<211>  8
<212>  PRT
<213>  unknown

<220>
<223>  CCK-8

<400>  73

<210>  74
<211>  12
<212>  PRT
<213>  unknown

<220>
<223>  CCK-12

<400>  74

Ile Ser Asp Arg Asp Tyr Met Gly Trp Met Asp Phe
1               5                   10

<210>  75
<211>  33
<212>  PRT
<213>  unknown

<220>
<223>  CCK-33

<400>  75

Lys Ala Pro Ser Gly Arg Met Ser Ile Val Lys Asn Leu Gln Asn Leu
1               5                   10                  15

Asp Pro Ser His Arg Ile Ser Asp Arg Asp Tyr Met Gly Trp Met Asp
            20                  25                  30

Phe

<210>  76
<211>  39
<212>  PRT
<213>  unknown

<220>
<223>  CCK-39

<400>  76

Tyr Ile Gln Gln Ala Arg Lys Ala Pro Ser Gly Arg Met Ser Ile Val
1               5                   10                  15

Lys Asn Leu Gln Asn Leu Asp Pro Ser His Arg Ile Ser Asp Arg Asp
            20                  25                  30

Tyr Met Gly Trp Met Asp Phe
        35

<210>  77
<211>  58
<212>  PRT
<213>  unknown

<220>
<223>  CCK-58

<400>  77

Val Ser Gln Arg Thr Asp Gly Glu Ser Arg Ala His Leu Gly Ala Leu
1               5                   10                  15

Leu Ala Arg Tyr Ile Gln Gln Ala Arg Lys Ala Pro Ser Gly Arg Met
            20                  25                  30

Ser Ile Val Lys Asn Leu Gln Asn Leu Asp Pro Ser His Arg Ile Ser
        35                  40                  45

Asp Arg Asp Tyr Met Gly Trp Met Asp Phe

50                  55


<210>  78
<211>  95
<212>  PRT
<213>  unknown

<220>
<223>  Cholecystokinin

<400>  78

Gln Pro Val Pro Pro Ala Asp Pro Ala Gly Ser Gly Leu Gln Arg Ala
1               5                   10                  15


Glu Glu Ala Pro Arg Arg Gln Leu Arg Val Ser Gln Arg Thr Asp Gly
            20                  25                  30


Glu Ser Arg Ala His Leu Gly Ala Leu Leu Ala Arg Tyr Ile Gln Gln
        35                  40                  45


Ala Arg Lys Ala Pro Ser Gly Arg Met Ser Ile Val Lys Asn Leu Gln
    50                  55                  60


Asn Leu Asp Pro Ser His Arg Ile Ser Asp Arg Asp Tyr Met Gly Trp
65                  70                  75                  80


Met Asp Phe Gly Arg Arg Ser Ala Glu Glu Tyr Glu Tyr Pro Ser
                85                  90                  95


<210>  79
<211>  27
<212>  PRT
<213>  unknown

<220>
<223>  Secretin

<400>  79

His Ser Asp Gly Thr Phe Thr Ser Glu Leu Ser Arg Leu Arg Glu Gly
1               5                   10                  15


Ala Arg Leu Gln Arg Leu Leu Gln Gly Leu Val
            20                  25


<210>  80
<211>  22
<212>  PRT
<213>  unknown

<220>
<223>  motilin

<400>  80

Phe Val Pro Ile Phe Thr Tyr Gly Glu Leu Gln Arg Met Gln Glu Lys
1               5                   10                  15

Glu Arg Asn Lys Gly Gln
            20

<210>  81
<211>  28
<212>  PRT
<213>  unknown

<220>
<223>  vasoactive intestinal peptide VIP

<400>  81

His Ser Asp Ala Val Phe Thr Asp Asn Tyr Thr Arg Leu Arg Lys Gln
1               5                   10                  15

Met Ala Val Lys Lys Tyr Leu Asn Ser Ile Leu Asn
            20                  25

<210>  82
<211>  34
<212>  PRT
<213>  unknown

<220>
<223>  PYY

<400>  82

Ile Lys Pro Glu Ala Pro Gly Glu Asp Ala Ser Pro Glu Glu Leu Asn
1               5                   10                  15

Arg Tyr Tyr Ala Ser Leu Arg His Tyr Leu Asn Leu Val Thr Arg Gln
            20                  25                  30

Arg Tyr

<210>  83
<211>  36
<212>  PRT
<213>  unknown

<220>
<223>  pancreatic hormone

<400>  83

Ala Pro Leu Glu Pro Val Tyr Pro Gly Asp Asn Ala Thr Pro Glu Gln
1               5                   10                  15

Met Ala Gln Tyr Ala Ala Asp Leu Arg Arg Tyr Ile Asn Met Leu Thr
            20                  25                  30

Arg Pro Arg Tyr
        35

<210>  84
<211>  20
<212>  PRT

<213>  unknown

<220>
<223>  pancreatic icosapeptide

<400>  84

His Lys Glu Asp Thr Leu Ala Phe Ser Glu Trp Gly Ser Pro His Ala
1               5                   10                  15

Ala Val Pro Arg
            20

<210>  85
<211>  28
<212>  PRT
<213>  unknown

<220>
<223>  ghrelin-28

<400>  85

Gly Ser Ser Phe Leu Ser Pro Glu His Gln Arg Val Gln Gln Arg Lys
1               5                   10                  15

Glu Ser Lys Lys Pro Pro Ala Lys Leu Gln Pro Arg
            20                  25

<210>  86
<211>  29
<212>  PRT
<213>  unknown

<220>
<223>  glucagon

<400>  86

His Ser Gln Gly Thr Phe Thr Ser Asp Tyr Ser Lys Tyr Leu Asp Ser
1               5                   10                  15

Arg Arg Ala Gln Asp Phe Val Gln Trp Leu Met Asn Thr
            20                  25

<210>  87
<211>  37
<212>  PRT
<213>  unknown

<220>
<223>  glucagon-like peptide (GLP-1)

<400>  87

His Asp Glu Phe Glu Arg His Ala Glu Gly Thr Phe Thr Ser Asp Val
1               5                   10                  15

Ser Ser Tyr Leu Glu Gly Gln Ala Ala Lys Glu Phe Ile Ala Trp Leu
            20                  25                  30

Val Lys Gly Arg Gly
        35

<210>  88
<211>  33
<212>  PRT
<213>  unknown

<220>
<223>  glucagon-like peptide GLP-2

<400>  88

His Ala Asp Gly Ser Phe Ser Asp Glu Met Asn Thr Ile Leu Asp Asn
1               5                   10                  15

Leu Ala Ala Arg Asp Phe Ile Asn Trp Leu Ile Gln Thr Lys Ile Thr
            20                  25                  30

Asp

<210>  89
<211>  69
<212>  PRT
<213>  unknown

<220>
<223>  glicentin

<400>  89

Arg Ser Leu Gln Asp Thr Glu Glu Lys Ser Arg Ser Phe Ser Ala Ser
1               5                   10                  15

Gln Ala Asp Pro Leu Ser Asp Pro Asp Gln Met Asn Glu Asp Lys Arg
            20                  25                  30

His Ser Gln Gly Thr Phe Thr Ser Asp Tyr Ser Lys Tyr Leu Asp Ser
        35                  40                  45

Arg Arg Ala Gln Asp Phe Val Gln Trp Leu Met Asn Thr Lys Arg Asn
    50                  55                  60

Arg Asn Asn Ile Ala
65

<210>  90
<211>  42
<212>  PRT
<213>  unknown

<220>
<223>  gastric inhibitory polypeptide / Glucose dependent
       insulinotrophic polypeptide (GIP)

<400>  90

Tyr Ala Glu Gly Thr Phe Ile Ser Asp Tyr Ser Ile Ala Met Asp Lys
1               5                   10                  15

Ile His Gln Gln Asp Phe Val Asn Trp Leu Leu Ala Gln Lys Gly Lys
            20                  25                  30

Lys Asn Asp Trp Lys His Asn Ile Thr Gln
        35                  40

<210>  91
<211>  37
<212>  PRT
<213>  unknown

<220>
<223>  amylin

<400>  91

<210>  92
<211>  29
<212>  PRT
<213>  unknown

<220>
<223>  neutrophil defensin-2

<400>  92

Cys Tyr Cys Arg Ile Pro Ala Cys Ile Ala Gly Glu Arg Arg Tyr Gly
1               5                   10                  15

Thr Cys Ile Tyr Gln Gly Arg Leu Trp Ala Phe Cys Cys
            20                  25

<210>  93
<211>  44
<212>  PRT
<213>  unknown

<220>
<223>  somatomedin B

<400>  93

Asp Gln Glu Ser Cys Lys Gly Arg Cys Thr Glu Gly Phe Asn Val Asp
1               5                   10                  15

Lys Lys Cys Gln Cys Asp Glu Leu Cys Ser Tyr Tyr Gln Ser Cys Cys
            20                  25                  30

Thr Asp Tyr Thr Ala Glu Cys Lys Pro Gln Val Thr

35                  40

<210>  94
<211>  27
<212>  PRT
<213>  unknown

<220>
<223>  histadin-2

<400>  94

Arg Lys Phe His Glu Lys His His Ser His Arg Glu Phe Pro Phe Tyr
1               5                   10                  15

Gly Asp Tyr Gly Ser Asn Tyr Leu Tyr Asp Asn
            20                  25

<210>  95
<211>  43
<212>  PRT
<213>  unknown

<220>
<223>  beta defensin 126

<400>  95

Asn Trp Tyr Val Lys Lys Cys Leu Asn Asp Val Gly Ile Cys Lys Lys
1               5                   10                  15

Lys Cys Lys Pro Glu Glu Met His Val Lys Asn Gly Trp Ala Met Cys
            20                  25                  30

Gly Lys Gln Arg Asp Cys Cys Val Pro Ala Asp
        35                  40

<210>  96
<211>  146
<212>  PRT
<213>  unknown

<220>
<223>  leptin

<400>  96

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145